# FASCICULE DE BREVET EUROPEEN

(11) **EP 2 007 769 B1**
(45) Date de publication et mention de la délivrance du brevet: **15.06.2011**
(21) Numéro de dépôt: 07731259.3
(22) Date de dépôt: 06.04.2007
(51) Int. Cl.: C07D 493/22, A61K 31/357, A61P 35/00

(54) **DIMERES DE DERIVES D'ARTEMISININE, LEUR PRÉPARATION ET LEUR APPLICATION EN THÉRAPEUTIQUE**
DIMERE VON ARTEMISININDERIVATEN, DEREN HERSTELLUNG UND DEREN THERAPEUTISCHE VERWENDUNG
DIMERS OF ARTEMISININ DERIVATIVES, PREPARATION THEREOF AND THERAPEUTIC USE THEREOF

(30) Priorité: 11.04.2006 FR 0603209
(43) Date de publication de la demande: 31.12.2008
(73) Titulaire: Sanofi-Aventis, 75013 Paris (FR)
(72) Inventeur: COMMERCON, Alain, 75013 Paris (FR); ZHANG, Jidong, 75013 Paris (FR); HITTINGER, Augustin, 75013 Paris (FR)
(74) Mandataire: Bouvet, Philippe
(86) Numéro de dépôt international: PCT/FR2007/000585
(87) Numéro de publication internationale: WO 2007/116135

(56) Documents cités:
- US-A1- 2005 038 024
- US-B2- 6 790 863
- POSNER G. H. AND ALL: "Trioxane Dimers Have potent Antimalarial, Antiproliferative and Antitumor Activities In Vitro" BIOORGANIC AND MEDICINAL CHEMISTRY, vol. 5, no. 7, 1997, pages 1257-1265, XP002409018

## Description

La présente invention se rapporte à des dimères de dérivés d'artémisinine, à leur préparation et à leur application en thérapeutique.

Plus particulièrement, l'invention se rapporte à des dimères de dérivés d'artémisinine présentant une activité anticancéreuse, et en particulier une activité inhibitrice de la prolifération cellulaire,

A ce jour, la plupart des composés commerciaux utilisés en chimiothérapie posent des problème importants d'effets secondaires, de tolérance par les patients ou encore de résistance. Ainsi, il y a un besoin important pour de nouvelles classes de composés susceptible d'agir en tant qu'agents anti-cancéreux.

Parmi les produits naturels, l'artémisinine est une endoperoxide sesquiterpene qui a été isolé en 1971 de la plante *Artemisia annua* et présente des propriétés antimalariques. Certains dérivés simples tels que la dihydroartémisinine ou l'artéméther ont été préparés et présentent également des propriétés anti-malariques. En plus de cette activité, il a été démontré que certains dérivés et dimères de l'artémisinine présentent des propriétés anti-cancéreuses (J. Med. Chem. 2003, 46, 987-994 , brevet US 6,790,863).

Le problème que se propose de résoudre la présente invention est d'obtenir de nouveaux produits sous forme de digères d'artémisinine présentant une activité anti-cancéreuse.

La présente invention a pour objet les produits tels que définis à l'une des revendications 1 à 9.

### Art antérieur

Aucun de ces digères de l'art antérieur (J. Med. Chem. 2003, 46, 987-S94 ; brevet US 8,790,863) n'est substitué par un groupe B₁ ou B₂ tel que décrit ci-dessus selon la présente invention.

US 6790863 décrit des dimères d'artémisine ne comportant pas de substituant -CF₃ et présentant un motif A différent.

L'article « Trioxane dimers have potent antimalarial antiproliferative and antitumor activités in vitro» Bioorganic and medicinal chemistry, Vol.5, N°7, 1997. pages 1257-1265 décrit des dimères ne comportant pas de substituant -CF₃. Ce document est relatif de plus à un domaine technique différent, celui des composés anti-malaria.

US 2005/038024 est relatif à un domaine technique différent, celui des composés anti-mataria. De plus, il ne décrit ni ne suggère de dimères.

Parmi ces produits, des produits préférés sont ceux pour lesquels A est -S-, -SO- ou encore -SO₂- .

D'autres produits de formule générale (1) sont ceux pour lesquels A est -N(CH₃)-.

Selon la présente invention, n₁ et n₂ sont préférentiellement identiques et ont pour valeur 2, 3 ou 4.

D'autres produits de formule générale (1) sont ceux pour lesquels A est choisi parmi -NH-, N(CH₂-C(O)O-CH₂-CH₃)- ou -N(CH₂-COOH)-, et éventuellement caractérisé en ce que n₁ et n₂ sont identiques et ont pour valeur 2.

D'autres produits de formule générale (1) sont ceux pour lesquels A est choisi parmi (C₁-C₆)alcénylène ou époxyde, et éventuellement caractérisé en ce que n₁ et n₂ sont identiques et ont pour valeur 1.

D'autres produits de formule générale (1) sont ceux pour lesquels A est choisi parmi -NHCO- ou -1,2,3-triazole, et éventuellement caractérisé en ce que n₁ et n₂ sont différents et ont indépendamment pour valeur 1 ou 2.

Les produits de formule (1) peuvent comporter un ou plusieurs atomes de carbone asymétriques. Ils peuvent donc exister sous forme d'énantiomères ou de diastéréoisomères. Ces énantiomères, diastéreoisomères, ainsi que leurs mélanges, y compris les mélanges racémiques, font partie de l'invention.

Les produits de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolément des produits de formule (1) font également partie de l'invention.

Les produits de formule (I) peuvent également exister sous forme d'hydrates ou de solvats, à savoir sous forme d'associations ou de combinaisons avec une ou plusieurs molécules d'eau ou avec un solvant. De tels hydrates et solvates font également partie de l'invention.

Dans le cadre de la présente invention, on entend par :
- un atome d'halogène : un fluor, un chlore, un brome ou un iode ;
- un groupe alkyle : un groupe aliphatique saturé linéaire ou ramifié. A titre d'exemples, on peut citer les groupes méthyle, éthyle, propyle, isopropyle, butyle, isobutyle, tertbutyle, pentyle, etc ;
- un groupe cycloalkyle : un groupe alkyle cyclique. A titre d'exemples, on peut citer les groupes cyclopropyle, méthylcyclopropyle, cyclobutyle, cyclopentyle, cyclohexyle, etc ;

- un groupe fluoroalkyle : un groupe alkyle dont un ou plusieurs atomes d'hydrogène ont été substitués par un atome de fluor ;
- un groupe alcényle: un groupe aliphatique mono- ou poly-insaturé, linéaire ou ramifié, comprenant par exemple une ou deux insaturations éthyléniques;
- un groupe alcynyle: un groupe aliphatique mono- ou poly-insaturé, alinéaire ou ramifié, comprenant par exemple une ou deux insaturations acétyléniques.

Il est entendu que le groupe divalent A est susceptible d'être lié dans les deux sens possibles.

Conformément à la présente invention, les produits de formule générale (I) peuvent être préparés selon les méthodes conventionnelles de la chimie organique. Des exemples de synthèse sont illustrés dans les schémas 1 à 4 ci-dessous, dans lequel les produits de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

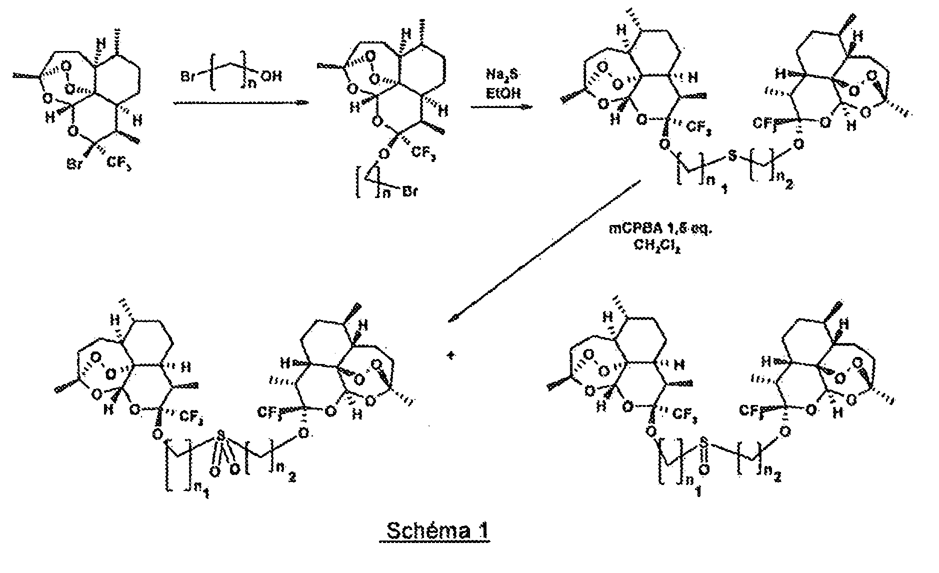

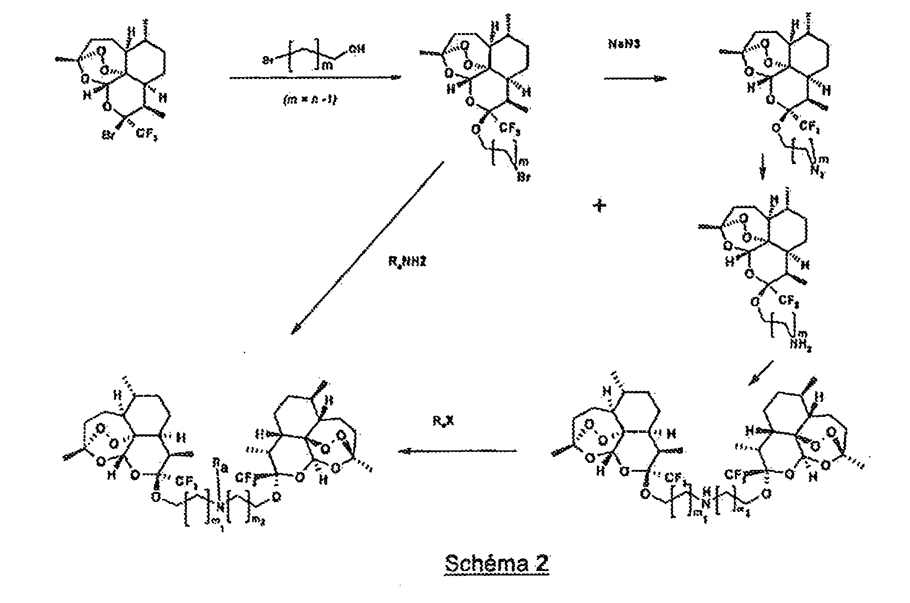

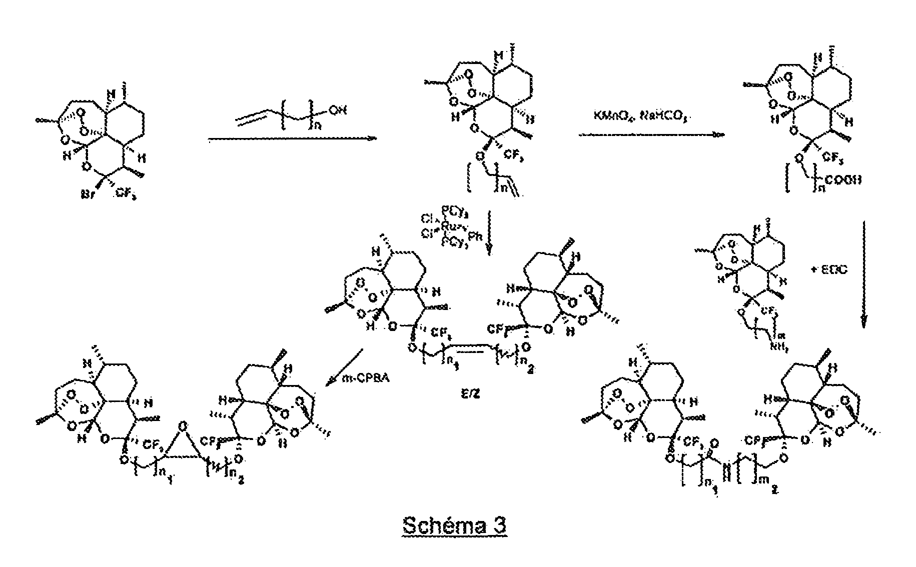

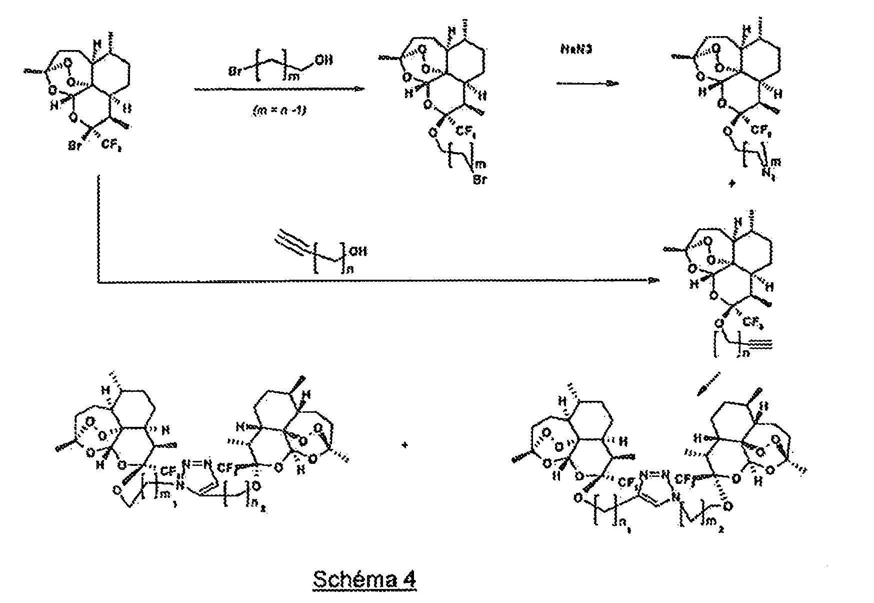

Un objet de la présente invention concerne un procédé de préparation d'un produit de formule général (I) caractérisé en ce que le produit de formule générale (III) suivante: 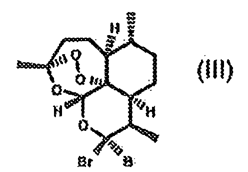 dans lequel B représente un substituant -CF₃ subit une substitution de l'atome de brome à l'aide d'un nucléophile tel qu'un bromo-alcool, pour donner un produit de formule générale (II) suivante 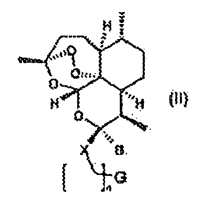 dans lequel X représente O n représente n₁ ou n₂, tel que défini précédemment, et dans lequel soit G représente un groupe partant tel qu'un atome de brome, puis ce produit de formule générale (II) subit une substitution nucléophile pour former un dimère de formule générale (I) ou un précurseur d'un produit de formule générale (I), soit G représente une fonction chimique F1, qui peut éventuellement être activé par une réaction de réduction ou d'oxydation, puis ce produit de formule générale (II) réagit avec un autre produit de formule (II) où G représente un groupe partant tel qu'un atome de brome ou une fonction chimique F2 susceptible de réagir avec F1, pour former un dimère de formule générale (I) ou un précurseur d'un produit de formule générale (I).

Un objet de la présente invention concerne plus particulièrement les produits de formule générale (II) dans laquelle X est un atome d'oxygène, n a pour valeur 0,1, 2, 3 ou 4, B est un groupe trifluorométhyl*e et G représente un atome de brome, un groupe -N3, -NH2, acényle, alcynyle ou -COOH. Ces composés sont utiles en tant que produits intermédiaires pour la synthèse des produits de formule générale (I).

On entend par groupe partant, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc, Des exemples de groupes partants ainsi que des références pour leur préparation sont donnés dans « Advances in Organic Chemistry », J. March, 3rd édition, Wiley Interscience, p. 310-316,

Les exemples suivants décrivent la préparation de certains produits conformes à l'invention. Ces exemples ne sont pas limitatifs et ne font qu'illustrer la présente invention.

### Abréviations;

**°C** degré Celsius **CCM** chromatographie sur couche mince; δ déplacement chimique; **d** doublet; **dd** doublet de doublets ; **DMSO-d⁶** diméthylsulfoxyde deutéré ; **dt** doublet de triplets ; **eq.** équivalent ; **ES+/-** electrospray (modes positif / négatif) ; **g** gramme; **h** heure ; **Hz** hertz ; **IC₅₀** constante d'inhibition à 80% d'activité; **J** constante de couplage ; **m** multiplet; **mg** miligramme: **MHz** mégahertz : **mL** millilitre ; **µL** microlitre ; **mm** millimètre ; **µm** micromètre **mmol** millimole ; **ppm** parties par million; **q** quadruplet ; **Rf** rapport frontal; **RMN ¹H** résonance magnétique nucléaire du proton ; **s** singulet ; **s1** singulet large **;** triplet ;; **U.V.** ultraviolet

### EX1: (3S,5aS.6R,8aS,9R,10R,12R,12aR,3'S,5'aS,6'R,8'aS,9'R, 10'R,12'R,12'aR)-10,10'-[thiobis(2,1-éthanediyloxy)]bis[décahydro-3,6,9-triméthyl-10-(trifiuorométhyl)-3,12-époxy-12H-pyrano[4,3-j-],2-benzodioxépine

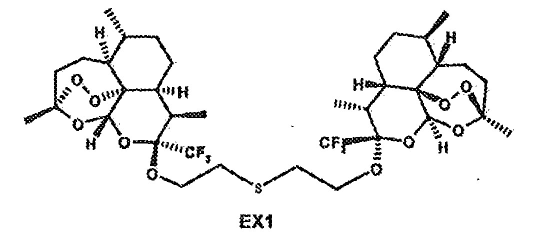

### a) Etape 1: Préparation de (3S,5aS,6R,8aS,9R,10R,12R,12aR,3'S,5'aS,6'R,8'aS,9'R,10'R,12'R,12'aR)- 10-(2-bromoéthoxy)decahydro-3,6,9-triméthyl-10-(trifluorométhyl)-3,12-époxy-12H-pyrano[4,3-j]-1,2-benzodioxépine 2

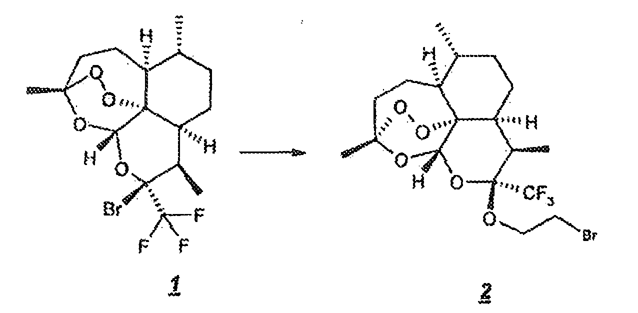

A une solution de 942 mg (2,27 mmol)de 3,12-epoxy-12*H-*pyrano[4,3*-j*]-1,2-benzodioxepin, (3*S*,5a*S*,6*R*,8a*S*,9*R*,10*S*,12*R*,12a*R*)-10-(bromo)decahydro-3,6,9-trimethyl-10(trifluoromethyl)-3,12-époxy-12*H-*pyrano[4,3*-j*]-1,2-benzodioxépine 1 (préparé selon Org. Lett. 2002, 4, 757-759), dans 20 mL de dichlorométhane, sont ajoutés successivement à température ambiante 1,2 ml d'héxafluoropropanol (5 éq.), puis 1,6 mL de bromo-2-éthanol (10 éq.). Le mélange réactionnel est ensuite agité à température ambiante pendant 2 heures 15 minutes, puis 10 mL d'une solution saturée de bicarbonate de sodium sont ajoutés. La phase organique est séchée sur sulfate de magnésium, et évaporée à sec sous pression réduite. Le résidu huileux obtenu est chromatographié sur gel de silice conditionné au préalable dans l'heptane, puis élué par un gradient linéaire de 0 à 100% du mélange B [(Heptane/Acétate d'éthyle), (90/10), (V/V)] dans A (Heptane). 217 mg (21%) du produit attendu 2 sont obtenus sous forme d'une huile,

Rf =0,45 dans le système (Heptane/Acétate d'éthyle), (90/10). (V/V)

ES.m/z=481 (MNa*)

RMN 1H à 400 MHz sur spectromètre BRUKER AVANCE DRX-400 avec les déplacements chimiques (δ en ppm) - dans le solvant chloroforme - d1 (CDCl3-d1) référencé à 7,27 à la température de 303K : 0,92 (m partiellement masqué, 1H) ; 0,96 (d, J = 6,5 Hz, 3H) ; 1,01 (d large, J = 7,5 Hz, 3H) ; de 1,21 à 1,58(m, 4H) ; 1,42 (s, 3H) ; de 1,65 à 2,08 (m,5H); 2,38 (m; 1H) ; 2,88 (m, 1H) ; de 3,45 à 3,57 (m, 2H) ; 4,02 (m, 1H) ; 4,17 (m, 1H); 5,57 (s, 1H).

### b) Etape 2: préparation l'EX1

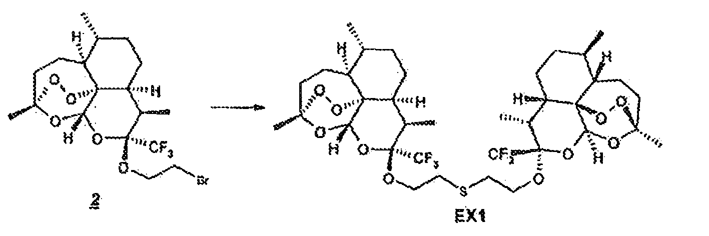

A une solution de 215 mg (0,47 mmol) de 2 dans 16 mL d'éthanol anhydre, sous atmosphère inerte d'argon à une température voisine de 20°C, sont ajoutés après 10 minutes 18 mg (0,234 mmol) de sulfure de sodium. L'agitation est maintenue à cette température pendant environ 190 heures. 20 mL d'une solution saturée en chlorure de sodium sont ensuite ajoutés, Le mélange est extrait par 3 x 20 mL d'acétate d'éthyle. Les phases organiques sont regroupées, lavées avec 20mL d'une solution saturée en NaCl, séchées sur sulfate de magnésium et évaporées à sec sous pression réduite. Le résidu huileux obtenu est chromatographie sur gel de silice conditionné au préalable dans l'heptane, puis élué par un gradient linéaire de 0 à 100% du mélange B [(Heptane/Acétate d'éthyle), (85/15), (V/V)] dans A (heptane) 60 mg (33%) du produit attendu **EX1** sont obtenus sous forme de meringue.

Rf = 0,20 dans le système (Heptane/Acétate d'éthyte), (90/10), (V/V)

ES : m/z=835 (M + HCOOH - H)

RMN 1H à 400 MHz sur spectromètre BRUKER AVANCE DRX-400 avec les déplacements chimiques (δ en ppm) - dans le solvant diméthylsulfoxide - d6 (DMSO-d6) référencé à 2,50 ppm à la température de 303K : 0,86 (m partiellement masqué, 2H); 0,90 (d, J = 6,5 Hz, 6H) ; 0,93 (d large, J = 7,5 Hz, 6H); 1,22 (m, 2H) ; de 1,27 à 1,60 (m, 8H) ; 1,31 (s, 6H) ; 1,66 (m, 2H); de 1,75 à 1,89 (m, 4H) ; 2,04 (m, 2H); 2,21 (m, 2H) : 2,67 (m, 2H); de 2,71 à 2,87 (m, 4H) ; 3,64 (m, 2H); 3,94 (m, 2H) ; 5,58 (s, 2H)

### EX2 :(3S,5aS,6R,8aS,9R,10R,12R,12aR,3'S,5'aS,6'R,8'aS,9'R,10'R,12'R;12'aR)-10,10'-[sulfinylbis(2,1-éthanediyloxy)]bis[décahydro-3,6,9-triméthyl-10-(trifluorométhyl)- 3,12-époxy-12H-pyrano[4,3-j]-1,2-benzodioxépine et EX3 : (3S,5aS,6R,8aS,9R,10R,12R,12aR,3'S,5'aS,6'R,8'aS,9'R,10'R,12'R,12'aR)-10,10'-[sulfonylbis(2,1-éthanediyloxy)]bis[décahydro-3,6,9-triméthyl-10-(trifluorométhyl)-3,12-époxy-12H-pyrano[4,3-j]-1,2-benzodioxépine

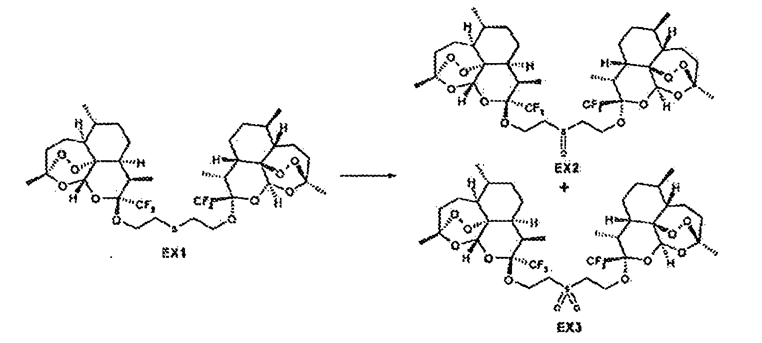

A une solution de 35 mg (0,044 mmol) de l'**EX1** dans 3 mL de dichlorométhane à une température voisine de 20°C, sont ajoutés lestement 16,4 mg (0,066 mmol) d'acide *meta*-chloroperbenzoique, L'agitation est maintenue à cette température pendant environ 3 heures puis 3 mL d'une solution saturée en bicarbonate de sodium sont ajoutés. Le mélange est extrait par 3 x 10 mL d'acétate d'éthyle, Les phases organiques sont regroupées, lavées avec 2x 10 mL d'une solution aqueuse saturée en chlorure de sodium, séchées sur sulfate de magnésium, filtrées et évaporées à sec sous pression réduite. Le résidu huileux obtenu est chromatographié sur gel de silice conditionné au préalable dans l'heptane, puis élué par un gradient linéaire de 0 à 100% d'acétate d'éthyle dans l'heptane. 14,5mg (40%) du produit **EX3** sont obtenus sous forme d'un solide blanc :

Rf = 0,60 dans le système (Heptane/Acétate d'éthyle), (50/50) (V/V)

Es m/z=867 (M + HCOOH - H)⁺

RMN 1H à 500 MHz sur spectromètre BRUKER AVANCE DRX-500 avec les déplacements chimiques (δ en ppm) - dans le solvant diméthylsulfoxide - d6 (DMSO-d6) référencé à 2,50 ppm à la température de 298K: 0,85 (m partiellement masqué, 2H); 0,89 (d, J = 6,5 Hz, 6H) 0,92 (d large, J = 7,5 Hz, 6H) ; 1,18 (m, 2H) ; 1.32 (si 6H) ; 1,36 (m, 2H) ; de 1,47 à 1.58 (m, 6H) ; 1,62 (m, 2H); 1,71 (m, 2H) ; 1,84 (m, 2H) : 2,04 (m, 2H) ; 2,21 (m, 2H) 2,68 (m, 2H) ; 3,48 (m, 2H) ; 3,59 (m, 2H) ; 3,83 (m, 2H); 4,19 (m, 2H) : 5,63 (s, 2H).

ainsi que 12,4 mg (35%) du produit **EX2** sous forme de meringue blanche ;

Rf = 0,22 dans le système (Heptane/Acétate d'éthyle), (50/50), (V/V)

ES : m/z=851(M + HCOOH - H)

RMN 1H à 500 MHz sur spectromètre BRUKER AVANCE DRX-500 avec les déplacements chimiques (δ en ppm) - dans le solvant diméthylsulfoxide - d6 (DMSO-d6) référencé à 2,50 ppm à la température de 298K (un mélange 50% ~50 % d'isomères) :0,85 (m partiellement masqué, 2H) ; 0,88 (d, J = 6,5 Hz, 3H) ; 0,90 . (d, J = 6,5 Hz, 3H) ; 0,93 (d large, J = 7.5 Hz, 6H) ; de 1,14 à 1,89 (m, 16H) ; 1,31 (s, 3H) ; 1,32 (s, 3H) ; 2,03 (m, 2H) ; 2,20 (m, 2H) ; 2,67 (m, 2H) ; 2,98 (m, 2H) ; 3,12 (m, 2H) ; 3,79 (m, 1H) 3,87 (m, 1H) de 4,11 à 4.20 (m, 2H) ; 5,58 (s, 1H) ; 5,61 (s, 1H).

### EX4 : (3S,5aS;6R,8aS,9R,10R,12R,12aR,3'S,5'aS,6'R,8'aS,9'R,10'R,12'R,12'aR)-10,10'-[thiobis(3,1-propanediyloxy)]bis[décahydro-3,6,9-triméthyl-10-(trifluorométhyl)-3,12-époxy-12H-pyrano[4,3-j]-1.2-benzodioxépine

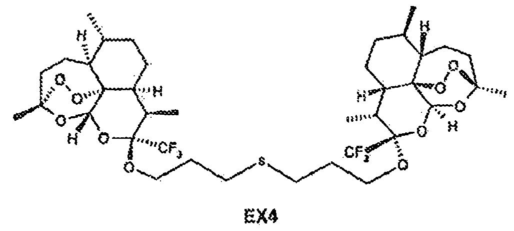

### a) Etape 1: Preparation du (3S,5aS,6R,8aS,9R,10R,12R,12aR,3'S,5'aS,6'R,8'aS,9'R,10'R,12'R,12'aR)- 10-(3-bromopropoxy)decahydro-3,6,9-triméthyl-10-(trifluorométhyl)-3,12-époxy-12H-pyrano[4,3-j]-1,2-bénzodioxépine 3

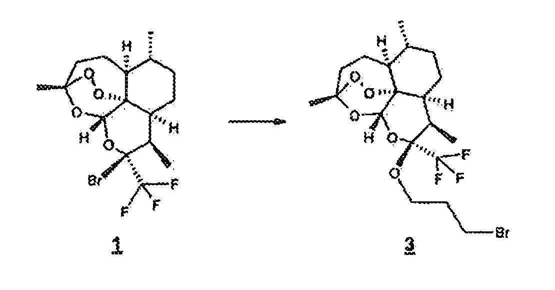

A une solution de 471 mg (1,14 mmol) de **1** dans 7 mL de dichlorométhane, sont ajoutés successivement à température ambiante 0,607 ml d'héxafluoropropanol (5 éq.), puis 1,03 mL de bromo-3-propanol (10 éq.). Le mélange réactionnel est ensuite agité à température ambiante pendant 2 heures 30 minutes, puis 5 mL d'une solution saturée de bicarbonate de sodium sont ajoutés, la phase organique est séchée sur sulfate de magnésium, filtrée et évaporée à sec sous pression réduite. Le résidu huileux obtenu est chromatographié sur gel de silice conditionnée au préalable dans l'heptane, puis élué par un gradient linéaire de 0 à 100% du mélange B [(Heptane/Acétate d'éthyle), (90/10), (V/V)] dans A (Heptane). 229 mg (43%) du produit attendu **3** sont obtenus sous forme d'une huile,

Rf = 0,38 dans le système (Heptane/Acétate d'éthyle), (90/10), (V/V)

IC m/z=490 (MNH₄)⁺; m/x=354 (m/z=490 - BrCH₂CH₂CH₂OH + 2H)*

RMN 1H à 400 MHz sur spectromètre BRUKER AVANCE DRX-400 avec les déplacements chimiques (δ en ppm) - dans le solvant chloroforme "- d1 (CDCl₃-d1) référencé à 7,27 ppm la température de 303K: 0,90 (m partiellement masqué, 1H) ; 0,97 (d, J = 6,5 Hz, 3H) ; 1,00 (d large, J = 7,5 Hz, 3H) ; de 1,22 à 1,54 (m, 4H) ; 1,44 (s, 3H) de 1,60 à 1,73 (m, 2H) : 1,82 (m, 1H) ; 1,91 (m, 1H) ; de 2,00 à 2,20 (m, 3H) ; 2,39 (m, 1H) : 2,87 (m, 1H) ; de 3,45 à 3,55 (m, 2H) ; 3,80 (m, 1H) 3,94 (m, 1H) ; 5,41 (s, 1H),

### b) Etape 2: préparation de l'EX4

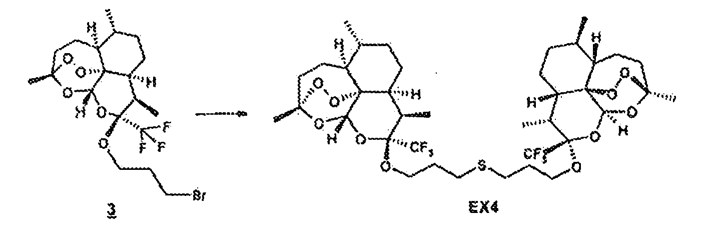

A une solution de 220 mg (0.46 mmol) du produit 3 dans 16 mL d'éthanol anhydre, sous atmosphère inerte d'argon à une température voisine de 20°C, sont ajoutés après 10 minutes 18,2 mg (0,23 mmol) de sulfure de sodium, l'agitation est maintenue à cette température pendant environ 80 heures puis 20 mL d'une solution saturée en chlorure de sodium sont ajoutés. Le mélange est extrait par 3 x 20 mL d'acétate d'éthyle. Les phases organiques sont regroupées, lavées avec 20mL d'une solution aqueuse saturée en chlorure de sodium, séchées sur sulfate de magnésium, filtrées et évaporées à sec sous pression réduite. Le résidu huileux obtenu est chromatographié sur gel de silice conditionné au préalable dans l'heptane, puis élué par un gradient linéaire de 0 à 100% du mélange B [(Heptane/Acétate d'éthyle), (85/15), (V/V)] dans A (Heptane). 103 mg (54%) du produit attendu **EX4** sont obtenus sous forme d'un solide blanc.

Rf = 0,20 dans le système (Heptane/Acétate d'éthyle), (90/10). (V/V)

ES : m/z=841 MNa⁺

RMN 1H 400 MHz sur spectromètre BRUKER AVANCE DRX-400 avec les déplacements chimiques (δ en ppm) - dans le solvant chloroforme - d1 (CDCl₃-d1) référencé à 7,27 à la température de 303K 0,93 (m partiellement masqué, 2H) ; 0,97 (d, J = 6,5 Hz, 8H) : 1,00 (d large, J = 7,5 Hz, 6H) ; de 1,22 à 1,38 (m, 4H); 1,43 (s, 6H) : de 1,44 à 1,55 (m, 4H) ; de 1,63 à 1,74 (m, 12H) ; 2,04 (m, 2H) ; 2,38 (m, 2H) ; de 2,51 à 2,66 (m, 4H) : 2,85 (m, 2H) ; 3,71 (m, 2H) ; 3,91 (m, 2H) ; 5,37 (s, 2H).

### EX5 :(3S,5aS,6R,8aS,9R,12R,12R,12aR,3'S,5'aS,6'R,8'S,9'R,10'R,12'R,12'aR)-10,10'-[sulfinylbis(3,1-propanediyloxy)]bis[décahydro-3,6,9-triméthyl-10-(trifluorométhy!)- 3,12-èpoxy-12H-pyrano[4,3-j]-1,2-benzodioxépine et EX6: (3S,5aS,6R,8aS,9R,10R,12R,12aR,3'S,5'aS,6'R,8'aS,9'R,10'R, 12'R,12'aR)- 10,10'-[sulfonylbis(3,1-propanediyloxy)]bis[décahydro-3,6,9-triméthyl-10-(trifluorométhyl)-3,12-èpoxy-12H-pyrano[4,3-j].1,2-benzodioxépine

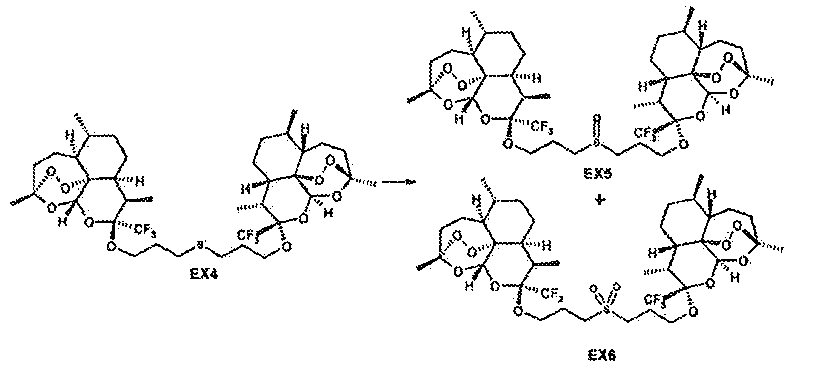

A une solution de 21,4 mg (0,026 mmol) de **l'EX4** dans 2 mL de dichlorométhane à une température voisine de 20°C, sont ajoutés lentement 9,1 mg (0,036 mmol) d'acide *meta* chloroperbenzoïque. L'agitation est maintenue à cette température pendant environ 3 heures puis 3 mL d'une solution saturée en bicarbonate de sodium sont ajoutés. Le mélange est extrait par 3 x 10 mL d'acétate d'éthyle. Les phases organiques sont regroupées, lavées avec 2 x 10 mL d'une solution aqueuse saturée en chlorure de sodium, séchées sur sulfate de magnésium, filtrées et évaporées à sec sous pression réduite. Le résidu huileux obtenu est chromatographié sur gel de silice conditionné au préalable dans l'heptane, puis élué par un gradient linéaire de 0 à 100% d'acétate d'éthyle dans l'heptane. 7,7 mg (34%) du produit **EX6** sont obtenus sous forme d'un solide blanc.

Rf = 0,68 dans le système (Heptane/Acétate d'éthyle), (50/50), (V/V)

ES : m/z=873 MNa⁺

RMN 1H à 400 MHz sur spectromètre BRUKER AVANCE DRX-400 avec les déplacements chimiques (δ en ppm) - dans le solvant chloroforme - d1 (CDCl3-d1) référencé à 7,27 à la température de 303K :0,92 (m partiellement masqué, 2H) ;;. 0,97 (d, J = 6,5 Hz, 6H) ; 1,00 (d large, J = 7,5 Hz, 6H) ; de 1,23 à 1,68 (m, 4H) ; 1,43 (s, 6H) ; de 1,44 à 1,72 (m, 8H) ; 1,83 (m, 2H) ; 1,92 (m, 2H) ; 2,05 (m, 2H) ; 2,12 (m, 4H) ; 2,38 (m, 2H) ; 2.87 (m, 2H) ; 3,00 (m, 2H) ; 3,13 (m, 2H) ; 3,75 (m, 2H) ; 3,97 (m, 2H) ; 5,34 (s, 2H).

ainsi que 8,5 mg (39%) du produit **EX5** sous forme de meringue blanche.

Rf = 0,20 dans le système (Heptane/Acétate d'éthyle), (50/50), (V/V)

ES : m/z=857 MNa⁺

RMN 1H à 400 MHz sur spectromètre BRUKER AVANCE DRX-400 avec les déplacements chimiques (δ en ppm) - dans le solvant chloroforme - d1 (CDCl3-d1) référencé à 7,27 à la température de 303K (un mélange 50%-50% d'isomères): 0,90 (m partiellement masqué, 2H) ; 0,97 (d, J = 6,5 Hz, 6H) ; 1,00 (d large, J = 7,5 Hz, 6H) ; de 1,24 à 1,36 (m, 4H) ; 1,43 (s, 6H) ; de 1,45 à 1,72 (m, 8H) ; 1,82 (m, 2H) ; 1,92 (m, 2H) ; de 2,00 à 2,11 (m, 6H) ; 2,38 (m, 2H) ; de 2,68 à 2,82 (m, 4H) ; 2,86 (m, 2H) ; 3,77 (m, 2H) ; 3,96 (m, 2H) ; 5,33 (s, 1H) ; 5,35 (s, 1H).

### EX7: (3S,5aS,6R,8aS,9R,10R,12R,12aR,3'S,5'aS,6'R,8'aS,9'R,10'R,12'R,12'aR)-10,10'-[thiobis(4,1-butanediyloxy)]bis[décahydro-3,6,9-triméthyl-10-(trifluorométhyl)-3,12-époxy-12H-pyrano[4,3-j]-1,2-benzodioxépine

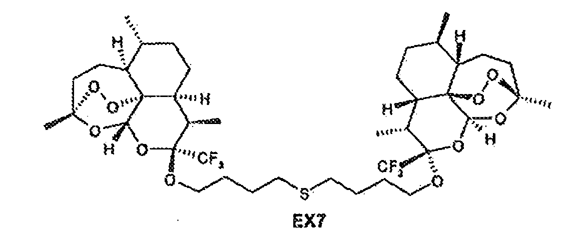

### a) Etape 1: preparation du (3S,5aS,6R,8aS,9R,10R,12R,12aR,3'S,5'aS,6'R,8'aS,9'R,10'R,12'R,12'aR)- 10-(4-bromobutoxy)decahydro-3,6,9-triméthyl-10-(trifluorométhyl)-3,12-époxy-12H-pyrano[4,3-j]-1,2-benzodioxépine, 4

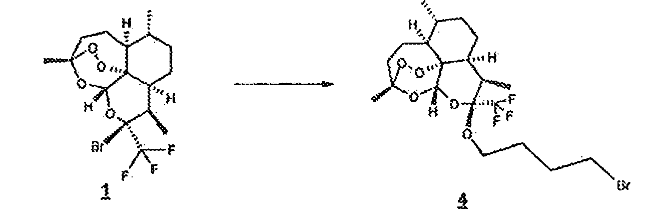

A une solution de 1,07 g (2,58 mmol) de 1 dans 15 mL de dichlorométhane, sont ajoutés successivement à température ambiante 1,36 ml d'héxafluoropropanol (5 éq.), puis 2,5 g de bromo-4-butanol (6,3 éq.). Le mélange réactionnel est ensuite agité à température ambiante pendant 3 heures, puis 6 mL d'une solution saturée de bicarbonate de sodium sont ajoutés. La phase organique est séchée sur sulfate de magnésium, filtrée et évaporée à sec sous pression réduite. Le résidu huileux obtenu est chromatographié sur gel de silice conditionné au préalable dans l'heptane, puis élué par un gradient linéaire de 0 à 100% du mélange B [(Heptane/Acétate d'éthyle), (90/10), (V/V)] dans A (Heptane). 90 mg (7%) du produit attendu **4** sont obtenus sous forme d'une huile.

Rf = 0,40 dans le système (Heptane/Acétate d'éthyle), (9/1), (V/V)

IC : m/z=504 MNH₄⁺

RMN 1H à 400 MHz sur spectromètre BRUKER AVANCE DRX-400 avec les déplacements chimiques (δ en ppm) - dans le solvant diméthylsulfoxide - d6 (DMSO-d6) référencé à 2,50 ppm à la température de 298K : 0,92 (m partiellement masqué, 1H) ; 0,97 (d,J = 6,0 Hz, 3H) ; 1,00 (d large, J = 7,5 Hz, 3H) ; de 1,05 à 1,62 (m partiellement masqué, 6H) ; 1,43 (s, 3H) ; de 1,65 à 2,10 (m, 7H) ; de 2,33 à 2,45 (m, 1H) ; 2,84 (m, 1H) ; de 3,40 à 3,51 (m, 2H) ; 3,63 (m, 1H) ; 3,88 (m, 1H) ; 5,32 (s, 1H).

### b) Etape 2: préparation de l'EX7

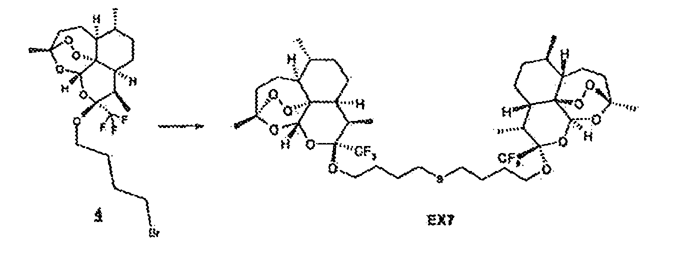

A une solution de 90 mg (0,19 mmol) de 4 dans 5 mL d'éthanol anhydre, sous atmosphère inerte d'argon à une température voisine de 20°C, sont ajoutés après 10 minutes 5,7 mg (0,073 mmol) de sulfure de sodium. L'agitation est maintenue à cette température pendant environ 42 heures puis 5 mL d'une solution saturée en chlorure de sodium sont ajoutés. Le mélange est extrait par 3 x 5 mL d'acétate d'éthyle. Les phases organiques sont regroupées, lavées avec 5 mL d'une solution aqueuse saturée en chlorure de sodium, séchées sur sulfate de magnésium, filtrées et évaporées à sec sous pression réduite. Le résidu huileux obtenu est chromatographié sur gel de silice conditionné au préalable dans l'heptane, puis élué par un gradient linéaire de 0 à 20% d'acétate d'éthyle dans l'heptane 33 mg (50%) du produit attendu **EX7** sont obtenus sous forme d'une meringue.

Rf = 0,20 dans le système (Heptane/Acétate d'éthyle), (9/1), (V/V)

ES ; m/z=891 (M + HCOOH - H)⁻

RMN 1H à 400 MHz sur spectromètre, BRUKER AVANCE DRX-400 avec les déplacements chimiques (δ en ppm) - dans le solvant diméthylsulfoxide - d6 (DMSO-d6) référencé à 2,50 ppm à la température de 298K : 0,93 (m partiellement masqué, 2H) ; 0,97 (d, J = 6,0 Hz, 6H) ; 1,00 (d large, J = 7,5 Hz, 6H) ; de 1,22 à 1,37 (m, 4H) ; 1,43 (s, 6H) ; 1.50 (m, 4H) ; de 1,62 à 1,86 (m, 14H) ; 1,91 (m, 2H) : 2,04 (m, 2H) ; 2,36 (m, 2H) ; 2.54 (m, 4H) ; 2,84 (m, 2H) ; 3,62 (m, 2H) 3,85 (m, 2H) ; 5,33 (s, 2H).

### EX8: 2-[[(3R,5aS,6R,8aS,9R,10R,12R,12aR)-decahydro-3,6,9-triméthyl-10-(trifluorométhyl)-3,12-époxy-12H-pyrano[4,3-j]-1,2-benzodioxépin-10-yl]oxy]-N-[2-[[(3R,5aS,6R,8aS,9R,10R,12R,12aR)-decahydro-3,6,9-triméthyl-10-(trifluorométhyl)-3,12-epoxy-12H-pyrano[4,3-j]-1,2-benzodioxépin-10-yl]oxy]éthyl]-N-méthyl- éthanamine

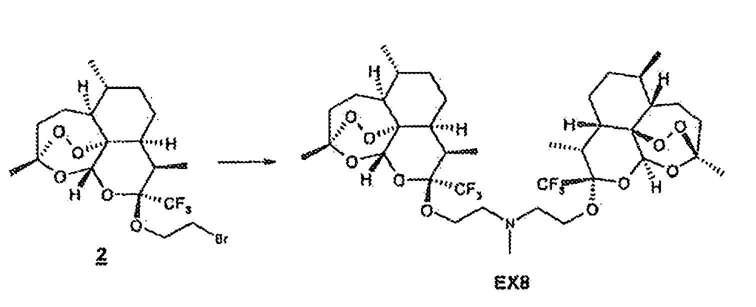

A une solution de 100 mg (0,218 mmol) du composé **2** dans 0,6 ml de tétrahydrofurane, sous atmosphère inerte d'argon à une température voisine de 20°C, sont ajoutés successivement 33 mg (0,218 mmol) d'iodure de sodium et 0,545 ml (1.09 mmol) d'une solution de méthylamine 2M dans le tétrahydrofurane. L'agitation est maintenue à 40°C pendant environ 20 heures. Le mélange réactionnel est repris par 3ml d'une solution aqueuse saturée en bicarbonate de sodium, puis extrait par 3x3 ml de dichlorométhane. Les phases organiques sont regroupées puis séchées sur sulfate de magnésium, filtrées et évaporées à sec sous pression réduite. Le résidu huileux obtenu est chromatographié sur gel de silice conditionné au préalable dans l'heptane, puis élué par un gradient de 0 à 30% d'acétate d'éthyle dans l'heptane. 15 mg (18%) du produit attendu EX8 sont obtenus sous forme d'un solide blanc.

Rf = 0,25 dans le système (Heptane/Acétate d'éthyle), (7/3), (V/V)

ES : m/z=788 MH⁺

RMN 1H à 400 MHz sur spectromètre BRUKER AVANCE DRX-400 avec les déplacements chimiques (δ en ppm) - dans le solvant chloroforme - d1 (CDCl3-d1) référencé à 7,27 à la température de 303K après addition d'une goutte d'acide acétique-d4-(CD3OD-d4) : 0,91 (m partiellement masqué, 2H) ; 0,95 (d, J = 6,5 Hz, 6H) ; 0,98 (d large, J = 7,0 Hz, 6H) ; 1,28 (m, 2H) ; de 1,33 à 1,63 (m, 8H) ; 1.39 (s, 6H) ; 1,69 (m, 2H) ; 1,78 (m, 2H) ; 1,89 (m, 2H) ; 2,02 (m partiellement masqué, 2H) ; 2,36 (m, 2H) ; 2,86 (m partiellement masqué, 2H) ; 2,89 (s, 3H) ; 3,38 (m, 4H) ; 4,19 (m, 4H) ; 5,38 (s, 2H).

### EX 9: 2-{[(3S,5aS,6R,8aS,9R,10R,12R,12aR)-3,6,9-triméthyl-10-(trifluorométhyl)décahydro-3,12-époxy[1,2]dioxépino[4,3-i]isochromén-10-yl]oxy}-N-(2-{[(3S,5aS,6R,8aS,9R,10R,12R,12aR)-3,6,9-triméthyl-10-(trifluorométhyl)décahydro-3,12-époxy[1,2]dioxépino[4,3-i]isochromén-10-yl]oxy}éthyl)éthanamine

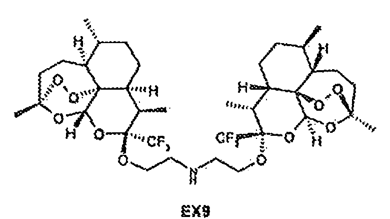

### a) étape 1 : préparation du (3R,5aS,6R,8aS,9R,10R,12R,12aR)-10-(2-azidoéthoxy)-3,6,9-triméthyl-10-(trifluorométhyl)décahydro-3,12-époxy[1,2]dioxépino[4,3-i]isochromène, 5

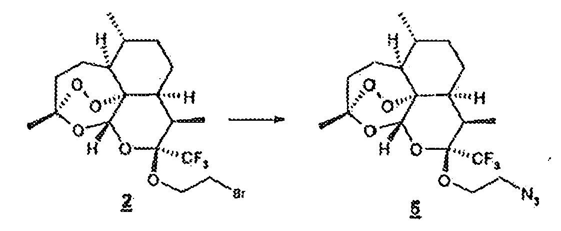

A une solution de 574 mg (1,25 mmol) du composé **2** dans 20 mL de diméthylformamide, sous atmosphère inerte d'argon à une température voisine de 20°C*,* sont ajoutés 162,5 mg (2,5 mmol) d'azoture de sodium. L'agitation est maintenue à une température voisine de 20°C pendant 3 heures. Le mélange réactionnel est repris par 60 mL d'eau distillée puis extrait par 2 x 100 mL d'acétate d'éthyle. La phase organique est lavée par 2 x 80 mL d'une solution aqueuse saturée en chlorure de sodium, séchée sur sulfate de magnésium, filtrée et évaporée à sec sous pression réduite. 527 mg (Rendement quantitatif) du produit attendu 6 sont obtenus sous forme d'une huile jaune.

Rf = 0,43 dans le système (Heptane/Acétate d'éthyle), (8/2), (V/V)

RMN ¹H (CDCl₃, 400MHz) δₚₚₘ : 5.48 (s, 1H) ; 3.97 (m, 1H) ; 3.78 (m, 1H) ; 3.48 (m, 2H) ; 2.88 (qu, 1H) ; 2,39 (td, 1H); 2.05 (dt, 1H) ; 197-1.88 (m, 1H) ; 1.87-1.77 (m, 2H) ; 1.76-1.68 (m, 1H); 1.53 (m, 1H) ; 1.50 (m, 1H) ; 1,43 (s, 3H) ; 1.39-1.25 (m, 2H) ; 1,03 (d, 3H) ; 1.01-0.89 (m, 1H) ; 0.98 (d, 3H).

### b) étape 2 : préparation du 2-([(3R,5aS,6R,8aS,9R,10R,12R,12aR)-3,6,9-triméthyl-10-(trifluorométhyl)décahydro-3,12-époxy[1,2]dioxépino[4,3-i]isochromén-10-yl]oxy}éthanamine, 6

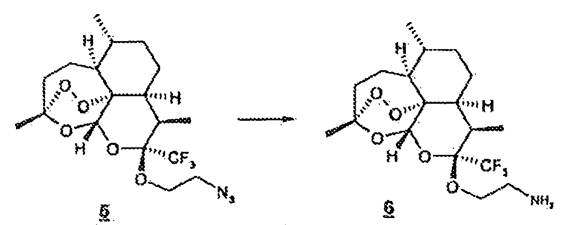

A une solution de 527 mg (1,25 mmol) du composé **5** dans 7 mL de tétrahydrofurane, sous atmosphère inerte d'argon à une température voisine de 20°C, sont ajoutés 328 mg (1,25 mmol) de triphénylphosphine. L'agitation est maintenue à une température voisine de 20°C pendant environ 24 heures. Le mélange réactionnel est repris par 1 mL d'eau distillée puis on poursuit l'agitation pendant environ 24 heures à la même température. Le mélange réactionnel est concentré sous vide et le résidu ainsi obtenu est repris par 5 mL de dichlorométhane, lavé par 2 mL d'une solution aqueuse saturée en bicarbonate de sodium, séchées sur sulfate de magnésium, filtrée et évaporée à sec sous pression réduite. Le résidu huileux obtenu est chromatographié sur gel de silice conditionné au préalable dans la dichlorométhane, puis élué par un gradient linéaire de 0 à 100% du mélange B [(Dichlorométhane/Méthanol), (90/10), (V/V)] dans A (Diclorométhane). 344 mg (70%) du produit attendu **6** sont obtenus sous forme d'une poudre blanche.

MS: ES⁺: [M+H]⁺ = 396.

Spectre RMN 1H à 400 MHz sur spectromètre BRUKER AVANCE DRX-400 avec les déplacements chimiques (δ en ppm) - dans le solvant chloroforme - d1 (CDCl3-d1) référencé à 7,27 à la température de 303K : 0,94 (m partiellement masqué, 1H) ; 0,97 (d, J = 6,5 Hz, 3H) ; 1,03 (d large, J = 7.0 Hz, 3H) ; 1,30 (m, 2H) ; de 1,40 à 1,56 (m, 2H) ; 1,42 (s, 3H) ; de 1,63 à 1,95 (m, 4H) ; 2,04 (m, 1H) ; 2,38 (m, ¹H) ; 2,45 (m étalé, 2H) ; 2,87 (m, 1H) ; 2,94 (m, 2H) ; 3,75 (m, 1H) ; 3,85 (m, 1H) ; 5,42 (s, 1H).

### c) étape 3 : préparation de EX9

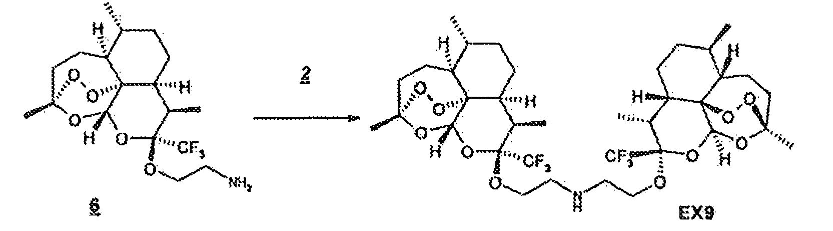

A une solution de 282 mg (0,534 mmol) du composé **2** dans 7 mL de diméthylformamide, sous atmosphère inerte d'argon à une température voisine de 20°C, sont ajoutés successivement 98 mg (0,593 mmol) d'iodure de potassium, 164 mg (1,19 mmol) de carbonate de potassium et 232 mg (0,587 mmol) du composé **6**. L'agitation est maintenue à 70°C pendant environ 7 heures. Le mélange réactionnel est concentré à sec sous pression réduire. Le résidu obtenu est repris par 10 mL de dichlorométhane, puis lavé par 6 mL d'eau distillée, la phase aqueuse est à nouveau extraite par 10 mL de dichlorométhane. Les phases organiques réunies sont lavées par 6 mL d'une solution aqueuse saturée en bicarbonate de sodium, puis séchées sur sulfate de magnésium, filtrées et évaporées à sec sous pression réduite. Le résidu obtenu est chromatographie sur gel de silice conditionné au préalable dans le mélange (Heptane/Acétate d'éthyle), (9/1), (V/V) puis élué par un gradient de 10 à 60% d'acétate d'éthyle dans l'heptane, 116 mg (28%) du produit attendu EX9 sont obtenus sous forme d'un solide jaune pâle.

Rf = 0,16 dans le système (Heptane/Acétate d'éthyle), (8/2), (V/V)

MS : ES⁺: [M+H]⁺ = 774.
ES⁻ : [M+HCOOH+H]⁺ = 818.

Spectre RMN 1H à 400 MHz sur spectromètre BRUKER AVANCE DRX-400 avec les déplacements chimiques (δ en ppm) - dans le solvant chloroforme - d1 (CDCl3-d1) référencé à 7,27 à la température de 303K : 0,92 (m partiellement masqué, 2H) ; 0,98 (d, J = 6,5 Hz, 6H) ; 1,00 (d large, J = 7,0 Hz, 6H) ; de 1,20 à 1,39 (m, 4H) ; 1,42 (s, 6H) ; de 1,40 à 1,83 (m, 11H) ; 1,90 (m, 2H) ; 2,03 (m, 2H) ; 2,38 (m, 2H) ; de 2,73 à 2,91 (m, 6H) ; 3,75 (m, 2H) ; 3,91 (m, 2H) ; 5,47 (s, 2H).

### EX 10 : N,N-bis(2-{[(3S,5aS,6R,8aS,9R,10R,12R,12aR)-3,6,9-triméthyl-10-(trifluorométhyl)décahydro-3,12-époxy[1,2]dioxépino[4,3-i]isochromén-10-yl]oxy}éthyl)glycinate d'éthyle

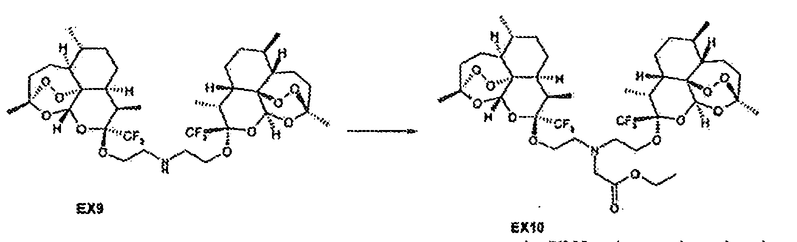

A une solution de 37 mg (0,047 mmol) du composé **EX9** dans 1 mL de diméthylformamide, sous atmosphère inerte d'argon à une température voisine de 20°C, sont ajoutés successivement 15 µL (0,133 mmol) de l'ester éthylique de l'acide bromoacétique, 8 mg (0,047 mmol) d'iodure de potassium, 10 mg ( 0,071 mmol) de carbonate de potassium. L'agitation est maintenue à 50°C pendant 1 heure. Le mélange réactionnel est concentré à sec sous pression réduite. Le résidu obtenu est repris par 5 mL d'acétate d'éthyle, puis lavé par 3 mL d'eau distillée la phase aqueuse est à nouveau extraite par 2x5 mL d'acétate d'éthyle. Les phases organiques réunies sont lavées par 2×5 mL d'une solution aqueuse saturée en chlorure de sodium, puis séchées sur sulfate de magnésium, filtrées et évaporées à sec sous pression réduite. Le résidu obtenu est chromatographié sur gel de silice conditionné au préalable puis élué dans le mélange(Heptane/Acétate d'éthyle), (80/20), (V/V), 30mg (74%) du produit attendu **EX10** sont obtenus sous forme d'une pâte incolore.

CCM Rf = 0,49 dans le système (Heptane/Acétate d'éthyle), (7/3), (V/V)

MS : ES⁺: [M+H]⁺ = 860.
ES⁻ : [M+HCOOH+H]⁺ = 904.

Spectre RMN 1N à 400 MHz sur spectromètre BRUKER AVANCE DRX-400 avec les déplacements chimiques (δ en ppm) - dans le solvant chloroforme - d1 (CDCl3-d1) référencé à 7,27 à la température de 303K: De 0,80 à 1,00 (m, 14H) ; de 1.19 à 1,38 (m partiellement masqué, 4H) ; 1,28 (t, J = 7,0 Hz, 3H) ; 1,40 (s, 6H) ; de 1,44 à 1,58 (m partiellement masqué, 4H) ; de 1,61 à 1,82 (m, 6H) ; 1,89 (m, 2H) ; 2,02 (m, 2H) ; 2,36 (m, 2H) ; de 2,70 à 2,90 (m, 4H) ; 3,02 (m, 2H) ; 3,40 (d, J = 17,5 Hz, 1H) ; 3,61 (m, 2H) ; 3,67 (d, J = 17,5 Hz, 1H) ; 3,98 (m, 2H) ; de 4,06 à 4,22 (m, 2H) ; 5,40 (s, 2H).

### EX 11 : N,N-bis(2-{[(3S,5aS,6R,8aS,9R,10R,12R,12aR)-3,6,9-triméthyl-10-(trifluorométhyl)décahydro-3,12-époxy[1,2]dioxépino[4,3-1]isochromén-10-yl]oxy}éthyl)glycine

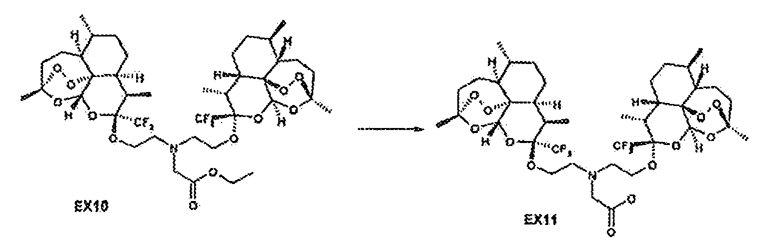

A une solution de 10 mg (0,011 mmol) du composé **EX10** dans 0,5 mL de méthanol, à une température voisine de 20°C, sont ajoutés 59 µL (0,059 mmol) d'une solution aqueuse de soude 1N. L'agitation est maintenue à la même température pendant environ 6,5 heures. Le mélange réactionnel est concentré à sec sous pression réduite. Le résidu obtenu est repris par 3 mL d'acétate d'éthyle, puis lavé par 1 mL d'une solution aqueuse saturée en chlorure de sodium. La phase aqueuse est à nouveau extraite par 3 mL d'acétate d'éthyle. Les phases organiques réunies séchées sur sulfate de magnésium, filtrées et évaporées à sec sous; pression réduite. Le résidu obtenu est chromatographié sur gel de silice conditionné au préalable puis élué dans le mélange (dichlorométhane/méthanol), (95/05), (V/V) 8 mg (81%) du produit attendu EX11 sont obtenus sous forme d'un solide incolore. MS : ES⁺ : [M+H]⁺ = 832.
ES⁻: [M-H]⁻ = 830.

Spectre RMN 1H à 300 MHz sur spectromètre BRUKER AVANCE DPX-400 avec les déplacements chimiques (δ en ppm) - dans le solvant chloroforme - d1 (CDCl3-d1) référencé à 7,27 à la température de 303K : 0,92 (m partiellement masqué, 2H) ; 0,97 (d, J = 6,5 Hz, 6H) ; 1,01 (d large, J = 7,0 Hz, 6H) ; de 1,20 à 1,38 (m partiellement masqué, 4H) ; 1,42 (s, 6H) ; de 1,44 à 1,75 (m, 8H) ; de 1,80 à 2,10 (m, 6H) ; 2,39 (m, 2H) ; de 2,80 à 3,02 (m, 6H) ; 3,38 (d, J = 17,5 Hz, 1H) ; 3,55 (d, J = 17,5 Hz, 1H) ; de 3,77 à 3,98 (m, 4H) ; 5,32 (s, 2H).

### EX 12 : 2-{[(3S,5aS,6R,8aS,9R,10R,12R,12aR)-3,6,9-triméthyl-10-(trifluorométhyl)décahydro-3,12-époxy[1,2]dioxépino[4,3-i]isochromén-10-yl]oxy}-N-(2-{[(35,5aS,6R,8aS,9R,10R,12R,12aR)-3,6,9-triméthyl-10-(trifluorométhyl)décahydro-3,12-époxy[1,2]dioxépino[4,3-i]isochromén-10-yl]oxy}éthyl)acétamide

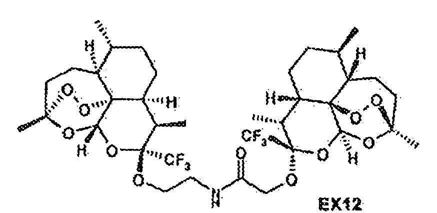

### a) étape 1 : préparation de acide {[(3R,5aS,6R,8aS,9R,10R,12R,12aR)-3,6,9-triméthyl-10-(trifluorométhyl)décahydro-3,12-époxy[1,2]dioxépino[4,3-i]isochromén-10-yl]oxy}acétique, 8

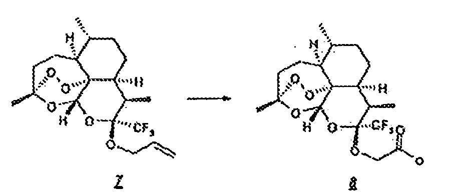

A une solution de 100 mg (0,255 mmol) de **7** (prépare selon WO2003035651), dans 2 mL d'acétone sont ajoutés successivement à température ambiante 117 mg (0,742 mmol) de permanganate de potassium puis 11 mg (0,127 mmol) de bicarbonate de sodium, Le mélange réactionnel est ensuite agité à température ambiante pendant 3 heures, puis 1 eq. d'une solution aqueuse 1 N d'acide chlorhydrique sont ajoutés. L'agitation est poursuivie à température ambiante pendant environ 18 heures. Le milieux réactionnel est filtré puis évaporé à sec sous pression réduite, le résidu obtenu est repris par 10 mL d'acétate d'éthyle. La phase organique est lavée par 3 mL d'eau distillée, la phase aqueuse est acidifiée par 2 mL d'une solution aqueuse 1 N d'acide chlorhydrique puis extraite par 10 mL d'acétate d'éthyle. La phase organique est séchée sur sulfate de magnésium, filtrée puis évaporé à sec sous pression réduite. 27mg (26%) du produit attendu **8** sont obtenus sous forme d'un solide blanc.

RMN ¹H (CDCl₃, 300MHz) δₚₚₘ : 5.47 (s, 1H) ; 4.67 (d, 1H) ; 4.26 (d, 1H,) ; 2.91 (qu, 1H) ; 238 (td, 1H) ; 2.19 (dq, 1H) ; 2.04 (dt, 1H) ; 1.90 (m, 1H) ; 1.77 (m, 2H) ; 1.70 (m, 1H) ; 1.59-1.48 (m, 1H) ; 1.48-1.45 (m, 1H) ; 1,42 (s, 3H); 1.40-1.24 (m, 2H) ; 1.05 (d, 3H) ; 0.98-0.87 (m, 1H) ; 0.96 (d, 3H).

### b) étape 2 : préparation de l'EX12

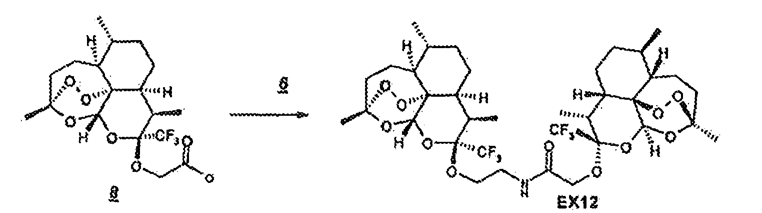

A une solution de 37 mg (0,09 mmol) du composé **8** dans 3 mL de dichlorométhane, sous atmosphère inerte d'argon à une température voisine de 20°C, sont ajoutés successivement 36 mg (0.27 mol) hydroxybenzotriaxol et 52 mg (0,27 mmol) de chlorhydrate de 1-(3-diméthylaminopropyl)-3-éthylcarbodiimide. L'agitation est maintenue à une température voisine de 20°C pendant environ 30 minutes, puis sont ajoutés 39 mg (0,099 mmol) du composé **6**. L'agitation est poursuivie à cette température pendant 1 heure. Le mélange réactionnel est repris par 5 mL d'eau distillée puis extrait par 3 x 20 mL d'acétate d'éthyle. La phase organique est lavés par 2 x 10 mL d'une solution aqueuse saturée en chlorure de sodium, séchées sur sulfate de magnésium, filtrée et évaporée à sec sous pression réduite. Le résidu obtenu est chromatographie sur gel de silice conditionné au préalable dans le mélange (Heptane/Acétate d'éthyle), (90/10), (V/V) puis élué par un gradient de 10 à 20% d'acétate d'éthyle dans l'heptane. 40 mg (56%) du produit attendu EX12 sont obtenus sous forme de gomme visqueuse.

MS : ES⁺: [M+H]⁺ = 788 ; [M+Na]⁺ = 810.
ES : [M-H]⁻ = 786 : [M+HCOOH+H]⁺ = 832.

Spectre RMN 1H à 400 MHz sur spectromètre BRUKER AVANCE DRX-400 avec les déplacements chimiques (δ en ppm) - dans le solvant chloroforme - d1 (CDCl3-d1) référencé à 7,27 à la température de 303K : De 0,87 à 1.03 (m, 11H) ; 1,08 (d large, 3H) ; de 1,21 à 1,40 (m, 4H) ; 1,43 (s, 6H) ; de 1,45 à 1,65 (m partiellement masqué, 6H) ; 1,69 (m, 2H) ; de 1,78 à 1,97 (m, 4H) ; 2,05 (m, 2H) ; de 2,32 à 2,43 (m partiellment masqué, 2H) ; de 2,82 à 2,98 (m, 2H) ; 3,37 (m, 1H) ; de 3,65 à 3,81 (m, 2H) ; 3,95 (m, 1H) ; 4,14 (d, J = 16,0 Hz, 1H) ; 4,43 (d, J = 16,0 Hz, 1H) ; 5,33 (s, 1H) ; 5,36 (s, 1H) ; 6,52 (m, 1H).

### EX 13: (3S,5aS,6R,8aS,9R,10R,12R,12aR,3'S,5aS,6'R,8a'S,9'R,10'R,12'R,12a'R)-10,10'-[(2E)-but-2-ène-1,4-diylbis(oxy)]bis[3,6,9-triméthyl-10-(trifluorométhyl)décahydro-3,12-époxy[1,2]dioxépino[4,3-i]isochromène] et EX14. (3S,5aS,6R,8aS,9R,10R,12R,12aR,3'S,5a'S,6'R,8a'S,9'R,10'R,12'R,12a'R)-10,10'-[(2Z)-but-2-ène-1,4-diylbis(oxy)]bis[3,6,9-triméthyl-10-(trifluorométhyl)décahydro-3,12-époxy[1,2]dioxépino[4,3-1]isochromène]

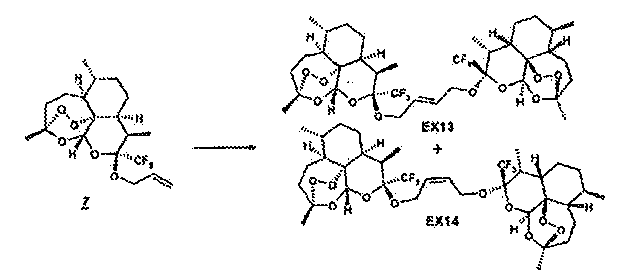

Une suspension de 200 mg (0,51 mmol) du composé **7** et 43 mg (0,051 mmol) de benzylidène-bis(tricyclohexylphosphine)dichlororuthénium dans 1,3 mL de dichlorométhane est agitée pendant environ 7 heures à une température voisine de 20°C. On ajoute une solution contenant 633 mg (5,10 mmol) de trishydroxyméthylphosphine et 1,43 mL (10,2 mmol) de triéthylamine, dans 3 mL de dichlorométhane. Une agitation énergique est maintenue à une température voisine de 20°C pendant environ 10 minutes, puis 6 mL d'eau sont ajoutés, l'agitation est poursuivie pendant 1 heure. La phase organique est lavée par 3 mL d'eau distillée, séchée sur sulfate de magnésium, filtrée et évaporé à sec sous pression réduite. Le résidu huileux obtenu est chromatographié sur gel de silice conditionné au préalable puis élué dans le mélange (Heptane/Acétate d'éthyle), (95/5), (V/V). 57,5 mg (38%) de l'isomère E **EX13** sont obtenus sous forme de cristaux blanc. 20 mg (10%) de l'isomère **Z EX14** sont obtenus sous forme de cristaux blanc.

### EX13 :

Rf = 0,41 dans le système (Heptane/Acétate d'éthyle), (8/2), (V/V)

IR: 983 cm⁻¹ (bande caractéristique CH=CH trans).

MS : ES⁺: [M+Na]⁺= 779.
ES⁻: [M+HCOOH+H]⁺ = 801.

RMN ¹H (CDCl₃, 300MHz) δₚₚₘ : 5.79 (t, 2H) ; 5.32 (s, 2H) ; 4.37 (dd, 2H) ; 4.19 (dd, 2H) ; 2.87 (qu, 2H) ; 2.39 (td, 2H) ; 2.04 (dt, 2H) ; 1.97.1.87 (m, 2H); 1.85-1.75 (m, 2H); 1.75-1.62 (m, 4H); 1.57.1.49 (m, 2H) ; 1.47 (m, 2H) ; 1.43 (s, 6H) ; 1.37-1.20 (m, 4H) ; 1.02 (d, 6H) ; 1.00-0.84 (m, 2H) ; 0.97 (d, 6H).

### EX14:

Rf = 0,47 dans le système (Heptane/Acétate d'éthyle), (8/2), (V/V)

MS: ES⁺: [M+NH₄]⁺ =774.

RMN ¹H (CDCl₃, 300MHz) δₚₚₘ : 5.67 (t, 2H) ; 5.31 (s, 2H) ; 4.42 (dd, 2H) ; 4.25 (dd, 2H) ; 2.85 (qu, 2H) ; 2.38 (td, 2H) ; 2.04 (dt, 2H) ; 1.96-1.84 (m, 2H) ;; 1.84-1.74 (m, 2H) ; 1.74-1.61 (m, 4H) ; 1.57-1.49 (m, 2H) ; 1.47 (m, 2H) ; 1.43 (s, 6H) ; 1.37-1.20 (m, 4H) ; 1.01 (d, 6H) ; 1.00-0.87 (m, 2H) ; 0.97 (d, 6H).

### EX15: (3S,5aS,6R,8aS,9R,10R,12R,12aR,3'S,5a'S,6'R,8a'S,9'R,10'R,12'R,12a'R)-10,10'[(2R,3R)-oxirane-2,3-diylbis(méthylèneoxy)]bis[3,6,9-triméthyl-10-(trifluorométhyl)décahydro-3,12-époxy[1,2]dioxépino[4,3-i]isochromènel

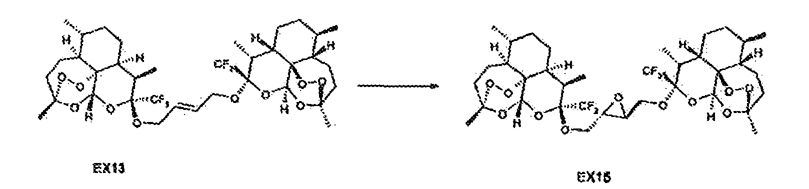

A une solution de 158 mg (0,209 mmol) du composé **EX13** dans 2,75 mL de dichlorométhane, à une température voisine de 20°C, sont ajoutés 103 mg (0,417 mmol) d'acide *meta*-chloroperbenzoïque, L'agitation est maintenue à cette température pendant environ 8 heures. Le mélange réactionnel est lavé successivement par 3x5 mL d'une solution aqueuse saturée en bicarbonate de sodium et par 13 mL d'une solution aqueuse saturée en chlorure de sodium. La phase organique est séchée sur sulfate de magnésium, filtrée et évaporée à sec sous pression réduite. Le résidu huileux obtenu est chromatographié sur gel de silice conditionné au préalable puis élué dans le mélange (Heptane/Acétate d'éthyle), (95/5), (V/V), 70 mg (43%) de l'un des deux l'isomères trans **EX15** sont obtenus sous forme de cristaux blanc.

MS: ES⁺ ; [M+Na]⁺ = 795.

Spectre RMN 1H à 400 MHz sur spectromètre BRUKER AVANCE DRX-400 avec les déplacements chimiques (δ en ppm) - dans le solvant chloroforme - d1 (CDCl3-d1) référencé à 7,27 à la température de 303K ; De 0,86 à 0,98 (m, 14H) ; de 1.20 à 1.39 (m, 4H) ; 1,42 (s, 6H) ; de 1,44 à 1,55 (m, 4H) ; de 1,62 à 1,77 (m, 6H) ; 1,89 (m, 2H) ; 2,04 (m, 2H) ; 2,37 (m, 2H) ; 2.85 (m, 2H) 3,11 (s large, 2H) ; 3,69 (d large, J = 12,5 Hz, 2H) ; 4,31 (d, J = 12,5 Hz, 2H) ; 5,39: (s, 2H).

### EX 16: (1-(2-{[(3S,5aS,6R,8aS,9R,10R,12R,12aR)-3,6,9-triméthyl-10-(trifluorométhyl)décahydro-3,12-époxy[1,2]dioxépino[4,3-i]isochromén-10-yl]oxy}éthyl)4-({[(3S,5aS,6R,8aS,9R,10R,12R,12aR)-3,6,9-triméthyl-10-(trifluorométhyl)décahydro-3,12-époxy[1,2]dioxépino[4,3-i]isochromén-10. yl]oxy}methyl}-1H-1,2,3-triazole et EX17: 1-(2-([(3S,5aS,6R,8aS,9R,10R,12R,12aR)-3,6,9-triméthyl-10-(trifluorométhyl)décahydro-3,12-époxy[1,2]dioxépino[4,31]isochromén-10-yl]oxy}éthyl)-5-({[(3S,5aS,6R,8aS,9R,10R,12R,12aR)-3,6,9-triméthyl-10-(trifluorométhyl)décahydro-3,12-époxy[1,2]dioxépino[4,3-i]isochromén-10-yl]oxy}éthyl)-1H-1,2,3-triazole

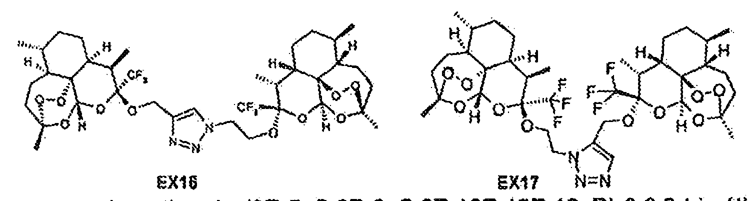

### a) étape 1 : préparation de (3R,5aS,6R,8aS,9R,10R,12R,12aR)-3,6,9-triméthyl-10-(prop-2-yn-1-yloxy)-10-(trifluoromethyl)décahydro-3, 12-époxy[1,2]dioxépino[4,3-i]isochromène, 9

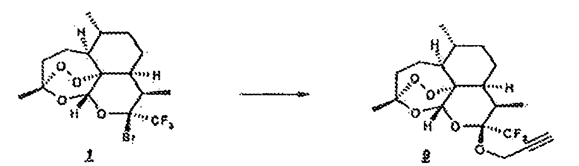

A une solution de 0,206 g (0,5 mmol) de 3,12-epoxy-12*H*-pyrano[4,3-*j*]-1,2-benzodioxepin, (3S,5a*S*,6*R*,8a*S*,9*R*,10*S*,12*R*,12a*R*)-10-(bromo)decahydro-3,6,9-trimethyl-10-(trifluoromethyl)- 3,12-époxy-12*H*-pyrano[4,3-*j*]-1,2-benzodioxépine **1** (préparé selon Org. Lett. 2002, 4, 757-759), dans 5 mL de dichlorométhane, sont ajoutés successivement à température ambiante 0,266 mL d'héxafluoropropanol (5 éq.), puis 0,289 mL d'alcool propargylique (10 éq.). Le mélange réactionnel est ensuite agité à température ambiante pendant 1 heure 15 minutes, puis 5 mL d'une solution saturée de bicarbonate de sodium sont ajoutés La phase organique est séchée sur sulfate de magnésium et évaporée à sec sous pression réduite. Le résidu huileux obtenu est chromatographié sur gel de silice conditionné au préalable dans l'heptane, puis élué par un gradient linéaire de 0 à 50% d'acétate d'éthyle dans l'héptane. 0,064 g (34%) du produit attendu **9** sont obtenus sous forme d'une huile.

Rf = 0,40 dans le système (Heptane/Acétate d'éthyle), (85/15), (V/V)

| | | |
|---|---|---|
| ES | m/z=391 | MH⁺ |
| | m/x=335 | MH⁺ - C₃H₄O |

Spectre RMN 1H à 400 MHz sur spectromètre BRUKER AVANCE DRX-400 avec les déplacements chimiques (δ en ppm) - dans le solvant diméthylsulfoxide - d6 (DMSO-d6) référencé à 2,50 ppm à la température de 303K : 0.88 (m partiellement masqué, 1H) ; 0,91 (d, J = 6,5 Hz, 3H) ; 0,95 (d large, J = 7,5 Hz, 3H) ; 1,23 (m, 1H) ; de 1,28 à 1,43 (m, 2H) ; 1,32 (s, 3H) ; de 1,50 à 1,63 (m, 2H) ; de 1,73 à 1,90 (m, 3H) ; 2,03 (m, 1H) ; 2,21 (m, 1H) ; 2,68 (m, 1H) ; 3,52 (t, J = 2,5 Hz, 1H) ; 4,30 (dd, J =2,5 et 16,0 Hz, 1H) ; 4,51 (dd, J = 2,5 et 16,0 Hz, 1H) ; 5,57 (s, 1H).

### b) étape 2: préparation de l'EX16 et de l'EX17

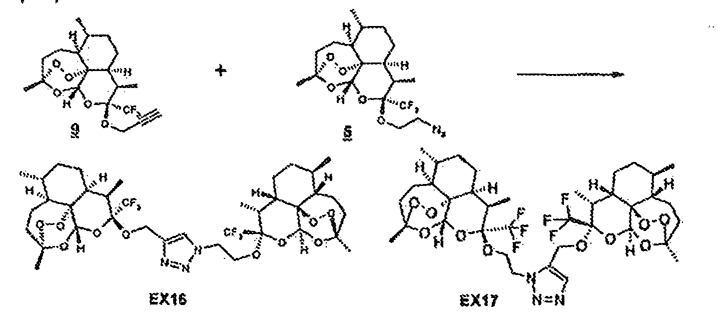

A une solution de 119 mg (0,305 mmol) du composé **9** dans 2,5 mL d'éthanol. à une température voisine de 20°C, sont ajoutés 257 mg (0,610 mmol) du composé 5. L'agitation est maintenue au reflux pendant environ 48 heures. Le mélange réactionnel est évaporé à sec au rotavapor et le résidu huileux obtenu est chromatographié sur gel de silice conditionné au préalable dans le dichlorométhane puis élué par un gradient linéaire de 0 à 100% du mélange B [(dichlorométhane/acétate d'éthyle), (96/4), (V/V)] dans A (dichlotométhane), 43mg (18%) du produit attendu **EX16** sont obtenus sous forme d'un solide blanc et 17mg (7%) du produit attendu **EX17** sont obtenus sous forme d'un solide blanc.

### EX16:

Rf = 0,26 dans le système (dichlorométhane/acétate d'éthyle), (96/4), (V/V).

| | | |
|---|---|---|
| ES | m/z=834 | MNa⁺ |
| | m/z=812 | MH⁺ |

Spectre RMN 1H à 400 MHz sur spectromètre BRUKER AVANCE DRX-400 avec les déplacements chimiques (δ en ppm) - dans le solvant chloroforme - d1 (CDCl3-d1) référencé à 7,27 à la température de 303K : De 0,75 à 1,70 (m partiellement masqué, 16H) ; 0,92 (d large, J = 7,0 Hz, 9H) ; 0,98 (d large, J = 7,0 Hz, 3H) ; 1,41 (s, 3H) ; 1,43 (s, 3H) ; 1,89 (m, 2H) ; 2,03 (m, 2H) ; 2,37 (m, 2H) ; 2,84 (m, 2H); 4,11 (m, 1H) ; 4,32 (m, 1H) ; de 4,40 à 4,80 (m, 2H) ; 4,71 (d, J = 12,5 Hz, 1H) ; 5,04 (d, J = 12, 5 Hz, 1H) ; 5,20 (s, 1H) 5,70 (s, 1H) ; 7,53 (s, 1H).

### EX17:

CCM Rf = 0,32 dans le système (dichlorométhane/acétate d'éthyle), (96/4), (V/V)

| | | |
|---|---|---|
| ES | m/z=834 | MNa⁺ |
| | m/z=812 | MH⁺ |

Spectre RMN 1H à 500 MHz sur spectromètre BRUKER AVANCE DRX-500 avec les déplacements chimiques (δ en ppm) - dans le solvant chloroforme - d1 (CDCl3-d1) référencé à 7,27 à la température de 303K: De 0,55 à 1,70 (m partiellement masqué, 16H) ; 0,90 (d large, J = 7,0 Hz, 9H) ; 1,01 (d large, J = 7,0 Hz, 3H) ; 1,39 (s, 3H) ; 1,44 (s, 3H) ; de 2,30 à 2,43 (m, 2H) ; de 1.97 à 2.09 (m, 2H) ; de 2,28 à 2,42 (m, 2H) ; 2,80 (m, 1H) ; 2,90 (m, 1H) ; 4,11 (m, 1H) ; de 4,42 à 4,63 (m, 3H) ; 4,40 (d, J = 13,0 Hz, 1H) ; 5,04 (d, J = 13,0 Hz, 1H) ; 5,11 (s, 1H) ; 5,24 (s, 1H) ; 7,61 (s, 1H).

### Activité antiproliférative des produits préparés:

Les produits selon l'invention ont fait l'objet d'essais pharmacologiques permettant de déterminer leur activité antiproliférative. Elle a été déterminée par mesure de l'inhibition de la prolifération cellulaire de cellules HCT116. Les cellules sont ensemencées dans un milieu de culture cellulaire à une concentration de 10 000 cellules par puits, dans 0.17 mL de milieu, et 20 µL de produit à tester, à différentes concentrations, et 10 µL de Thymidine [methyl-14C] (100 µCi/ml - activité spécifique 47,90 mCi/mmol; NEN Technologies référence NEC568 batch 3550-001) sont ajoutés, puis les cellules sont incubées à 37°C et 5% de CO₂.

Milieu utilisé pour la culture de cellules HCT116 : milieu DMEM 2 mM L-glutamine, 200 Ul/ml pénicilline, 200µg/ml streptomycine and 10% (V/V) Sérum de veau foetal (Life Technologies).

Après 96 heures, l'incorporation de ¹⁴C-thymidine est comptée dans un compteur à scintillation liquide 1450 Microbeta Wallac Trilux. Les résultats R sont exprimés en cpm (coups par minute) et convertis en pourcentage d'inhibition de croissance Gl% en faisant premièrement la soustraction de la moyenne du nombre de cpm des puits sans cellules B et en divisant ensuite par le nombre de cpm des puits des cellules non traitées C comprenant 20µL de milieu de dilution du produit contentant 1% d'éthanol. (GI % = (R - B) x 100/ C %).

Les valeurs d'IC50 sont calculées à l'aide de l'équation 205 du logiciel XLFit (IDBS company, UK) par analyse de régression non linéaire utilisant l'algorithme Marquardt (Donald W. MARQUARDT, J.Soc.industry.appl, vol 11, No. 2, June, 1963).

Les produits présentent une IC50 sur les cellules HCT116 généralement Inférieure à 10µM et de préférence inférieure à 100nM.

| Exemples | IC50 (nM) /HCT116 |
|---|---|
| **EX1** | 47 |
| **EX2** | 23 |
| **EX3** | 21 |
| **EX8** | 30 |

Les produits selon l'invention peuvent donc être utilisés pour la préparation de médicaments.

Ainsi, selon un autre de ses aspects, l'invention a pour objet des médicaments qui comprennent un produit de formule (I) ou un sel d'addition de ce dernier à un acide pharmaceutiquement acceptable, ou encore un hydrate ou un solvant.

Ces médicaments trouvent leur emploi en thérapeutique, notamment dans le traitement du cancer.

La présente invention concerne donc l'utilisation d'un produit de formule (I) pour la fabrication d'un médicament utile pour traiter un état pathologique et plus particulièrement l'utilisation d'un produit de formule (I) pour la fabrication d'un médicament utile pour traiter le cancer.

La présente invention concerne également l'utilisation d'un produit de formule (I) pour la fabrication d'un médicament utile pour traiter les pathologies où une néovascularisation ou angiogénèse se fait de façon inappropriée c'est-à-dire dans les cancers en général mais aussi dans des cancers particuliers tels que le sarcome de Kaposi ou l'hémoangiome infantile, et aussi dans l'arthrite rhumatoïde, l'osthéoarthrite et/ou ses douleurs associée, les maladies inflammatoires de l'intestin telle que la recto-colite hémorragique ou la maladie de Crohn's, les pathologies de l'oeil telle que la dégénérescence maculaire liée à l'âge, les rétinopathies diabétiques, l'inflammation chronique, le psoriasis.

L'angiogenèse est un processus de génération de nouveaux vaisseaux capillaires à partir des vaisseaux pré-existants. L'angiogenèse tumorale (formation de néovaisseaux sanguins), indispensable à la croissance tumorale, est également un des facteurs essentiels de la dissémination métastatique (Oncogene, 2003 May 19;22(20):3172-9; Nat Med. 1995 Jan;1(1):27-31.).

Selon un autre de ses aspects, la présente invention concerne des compositions pharmaceutiques comprenant, en tant que principe actif, un produit selon l'invention. Ces compositions pharmaceutiques contiennent une dose efficace d'au moins un produit selon l'invention ou un sel pharmaceutiquement acceptable, un hydrate ou solvant dudit produit, ainsi qu'au moins un excipient pharmaceutiquement acceptable

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intra-trachéale, intra-nasale, transdermique ou rectale, le principe actif de formule (I) ci-dessus, ou son sel, solvat ou hydrate, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intra-trachéale, intraoculaire, intra-nasale, par inhalation, les formes d'administration topique, transdermique, sous-cutanée, intramusculaire ou intraveineuse, les formes d'administration rectale et les implants, Pour l'application topique, on peut utiliser les produits selon invention dans des crèmes, gels, pommades ou lotions.

A titre d'exemple, une forme unitaire d'administration d'un produit selon l'invention sous forme de comprimé peut comprendre les composants suivants :

| | |
|---|---|
| Produit selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles: sont appropriés : de tels dosages ne sortent pas du cadre de l'invention. Selon la pratique habituelle, le dosage approprié à chaque patient est déterminé par le médecin selon le mode d'administration, le poids et la réponse dudit patient.

Les produits de la présente invention peuvent être administrés seuls ou en mélange avec d'autres anticancéreux. Parmi les associations possibles on peut citer:
- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmétamine
- les dérivés du platine comme notamment la cisplatine, le carboplatine ou l'oxaliplatine
- les agents antibiotiques comme notamment la bléomycine, la mitomycine, la dactinomycine
- les agents antimicrotubules comme notamment la vinblastine, la vincristine, la vindésine, la vinorelbine, les taxoïdes (paclitaxel et docétaxel)
- les anthracyclines comme notamment la doxorubicine, la daunorubicine, l'idarubicine, l'épirubicine, la mitoxantrone, la losoxantrone
- les inhibiteurs de topoisomérases des groupes I et II telles que l'étoposide, le teniposide, l'amsacrine, l'irinotecan, le topotecan et le tomudex
- les fluoropyrimidines telles que la 5-fluorouracile, l'UFT, la floxuridine
- les analogues de cytidine telles que la 5-azacytidine, la cytarabine, la gemcitabine, la 6-mercaptomurine, la 6-thioguanine
- les analogues d'adénosine tels que la pentostatine, la cytarabine ou le phosphate de fludarabine
- le méthotrexate et l'acide folinique
- les enzymes et produits divers tels que la L-asparaginase, l'hydroxyurée, l'acide trans-rétinoique, la suramine, la dexrazoxane, l'amifostine, l'herceptine ainsi que les hormones oestrogéniques, androgéniques
- les agents antivasculaires tels que les dérivés de la combretastatine par exemple la CA4P, des chalcones ou de la colchicine, par exemple le ZD6126, et leurs prodrogues.
- Les inhibiteurs de kinases tels que l'ertonilib ou l'imatinib
- Les agents biothérapeutiques comme les anticorps tels que le rituximab, le bevacizumab, le cetuximab; le trastuzumab ou l'alemtuzumab
- Les inhibiteurs du protéasome tel que le bortesomib

Il est également possible d'associer aux produits de la présente invention un traitement par des radiations. Ces traitements peuvent être administrés simultanément, séparément, séquentiellement. Le traitement sera adapté par le praticien en fonction du malade à traiter.

## Revendications

1. Produit anticancéreux de formule (I) : 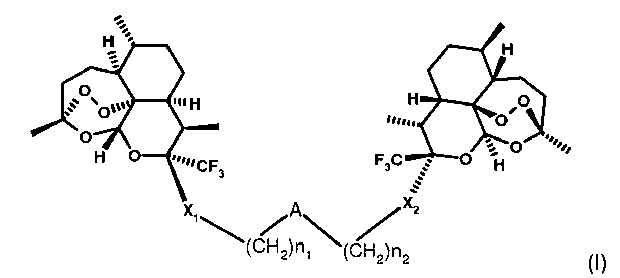 dans laquelle :
a). A est un groupe divalent choisi parmi -S-, -SO-, -SO₂-, -NH-, -N(CH₂-C(O)O-CH₂-CH₃)- ou -N(CH₂-COOH)-, époxyde, (C₁-C₆)alcénylène, -NHCO-, 1,2,3-triazole ;
b). X₁ et X₂ sont identiques et sont O ;
c). n₁ et n₂ sont identiques ou différents, et ont pour valeur 1, 2, 3 ou 4 ;
à l'état de base ou de sel d'addition à un acide, ainsi qu'à l'état d'hydrate ou de solvat.

2. Produit selon la revendication 1 **caractérisé en ce que** n₁ et n₂ sont identiques et ont pour valeur 2, 3 ou 4.

3. Produit selon la revendication 1 **caractérisé en ce que** A est choisi parmi -NH-, -N(CH₂-C(O)O-CH₂-CH₃)- ou -N(CH₂-COOH)- et que n₁ et n₂ sont identiques et ont pour valeur 2.

4. Produit selon la revendication 1 **caractérisé en ce que** A est choisi parmi (C₁-C₆)alcénylène ou époxyde et que n₁ et n₂ sont identiques et ont pour valeur 1.

5. Produit selon la revendication 1 **caractérisé en ce que** A est choisi parmi -NHCO- ou 1,2,3-triazole et que n₁ et n₂ sont différents et ont indépendemment pour valeur 1 ou 2.

6. Produit selon l'une quelconque des revendications 1 à 5, **caractérisé en ce que** le produit est sous forme
• non chirale, ou
• racémique, ou
• enrichie en un stéréo-isomère, ou
• enrichie en un énantiomère ;
et **en ce qu'**il est éventuellement salifié.

7. Produit selon la revendication 1 choisi dans la liste suivante :
• (3S,5aS,6R,8aS,9R,10R,12R,12aR,3'S,5'aS,6'R,8'aS,9'R,10'R,12'R,12'aR)-10,10'-[thiobis(2,1-ethanediyloxy)]bis[decahydro-3,6,9-trimethyl-10-(trifluoromethyl)-3,12-epoxy-12H-pyrano[4,3-j]-1,2-benzodioxepine;
• (3S,5aS,6R,8aS,9R,10R,12R,12aR,3'S,5'aS,6'R,8'aS,9'R,10'R,12'R,12'aR)-10,10'-[sulfinylbis(2,1-ethanediyloxy)]bis[decahydro-3,6,9-trimethyl-10-(trifluoromethyl)-3,12-epoxy-12H-pyrano[4,3-j]-1,2-benzodioxepine;
• (3S,5aS,6R,8aS,9R,10R,12R,12aR,3'S,5'aS,6'R,8'aS,9'R,10'R,12'R,12'aR)-10,10'-[sulfonylbis(2,1 -ethanediyloxy)]bis[decahydro-3,6,9-trimethyl-10-trifluoromethyl)-3,12-epoxy-12H-pyrano[4,3-j]-1,2-benzodioxepine;
• (3S,5aS,6R,8aS,9R,10R,12R,12aR,3'S,5'aS,6'R,8'aS,9'R,10'R,12'R,12'aR)-10,10'-[thiobis(3,1-propanediyloxy)]bis[decahydro-3,6,9-trimethyl-10-(trifluoromethyl)-3,-12-epoxy-12H-pyrano[4,3-j]-1,2-benzodioxepine;
• (3S,5aS,6R,8aS,9R,10R,12R,12aR,3'S,5'aS,6'R,8'aS,9'R,10'R,12'R,12'aR)-10,10'-[sulfinylbis(3,1-propanediyloxy)]bis[decahydro-3,6,9-trimethyl-10-(trifluoromethyl)-3,12-epoxy-12H-pyrano[4,3-j]-1,2-benzodioxepine;
• (3S,5aS,6R,8aS,9R,10R,12R,12aR,3'S,5'aS,6'R,8'aS,9'R,10'R,12'R,12'aR)-10,10'-[sulfonylbis(3,1-propanediyloxy)]bis[decahydro-3,6,9-trimethyl-10-(trifluoromethyl)-3,12-epoxy-12H-pyrano(4,3-j]-1,2-benzodioxepine;
• (3S,5aS,6R,8aS,9R,10R,12R,12aR,3'S,5'aS,6'R,8'aS,9'R,10'R,12'R,12'aR)-10,10'-[thiobis(4,1-butanediyloxy)]bis[decahydro-3,6,9-trimethyl-10-(trifluoromethyl)-3,12-epoxy-12H-pyrano[4,3-j]-1,2-benzodioxepine;
• 2-[[(3R,5aS,6R,8aS,9R,10R,12R,12aR)-decahydro-3,6,9-trimethyl-10-(trifluoromethyl)-3,12-epoxy-12H-pyrano[4,3-j]-1,2-benzodioxepin-1 0-yl]oxy]-N-[2-[[(3R,5aS,6R,8aS,9R,10R,12R,12aR)-decahydro-3,6,9-trimethyl-10-(trifluoromethyl)-3,12-epoxy-12H-pyrano[4,3-j]-1,2-benzodioxepin-1 0-yl]oxy]ethyl]-N-methylethanamine;
• 2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}-N-(2-{((3S,5aS,6R,8aS,9R,10 R,12R,12aR)-3,6,9-trimethyl-10-(trifluoromethyl)decahydro-3,12-epoxy[1,2]dioxepino[4,3-i]isochromen-10-yl]oxy}ethyl)ethanamine;
• Ethyl N,N-bis(2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}ethyl)glycinate;
• N,N-bis(2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}ethyl)glycine;
• 2-{[(3S,5aS,6R,8aS,9R,1 OR,12R,12aR)-3,6,9-trimethyl-10-(trifluoromethyl)decahydro-3,12-epoxy[1,2]dioxepino(4,3-i]isochromen-10-yl]oxy}-N-(2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}ethyl)acetamide;
• (3S,5aS,6R,8aS,9R,10R,12R,12aR,3'S,5'aS,6'R,8'aS,9'R,10'R,12'R,12'aR)-10,10'-[(2E)-but-2-ene-1,4-diylbis(oxy)]bis[3,6,9-trimethyl-10-(trifluoromethyl)-decahydro-3,12-epoxy[1,2]dioxepino[4,3-i]isochromene];
• (3S,5aS,6R,8aS,9R,10R,12R,12aR,3'S,5'aS,6'R,8'aS,9'R,10'R,12'R,12'aR)-10,10'-[(2Z)-but-2-ene-1,4-diylbis(oxy)]bis[3,6,9-trimethyl-10-(trifluoromethyl)-decahydro-3,12-epoxy[1,2]dioxepino[4,3-i]isochromene];
• (3S,5aS,6R,8aS,9R,10R,12R,12aR,3'S,5'aS,6'R,8'aS,9'R,10'R,12'R,12'aR)-10,10'-[(2R,3R)-oxirane-2,3-diylbis(methyleneoxy)]bis[3,6,9-trimethyl-10-(trifluoromethyl)decahydro-3,12-epoxy[1,2]dioxepino[4,3-i]isochromene];
• (1-(2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}ethyl)-4-({[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}methyl)-1H-1,2,3-triazole;
• 1-(2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}ethyl)-5-({[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}ethyl)-1H-1,2,3-triazole.

8. Produit selon la revendication 1 choisi dans la liste suivante :

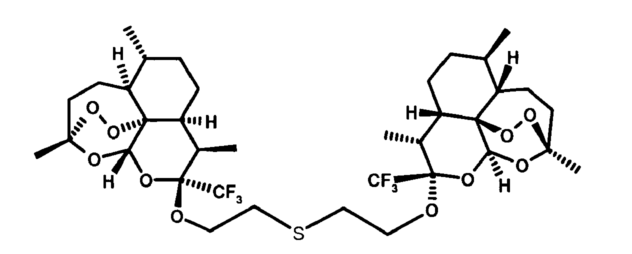

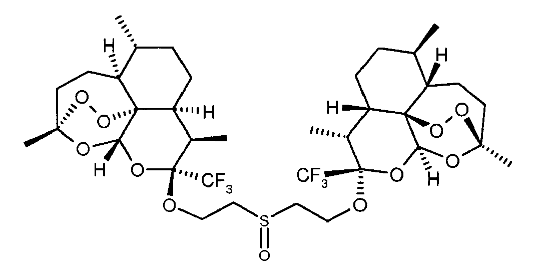

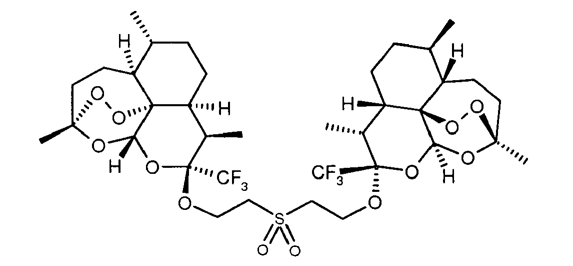

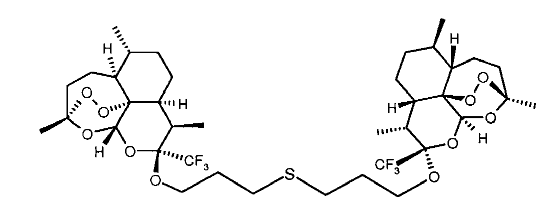

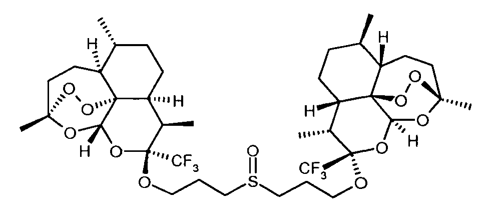

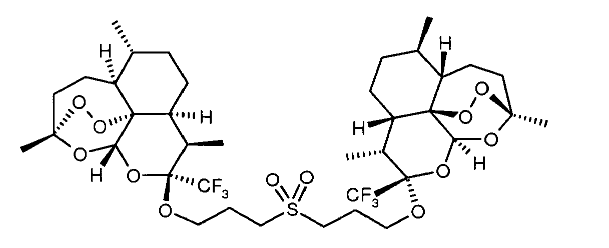

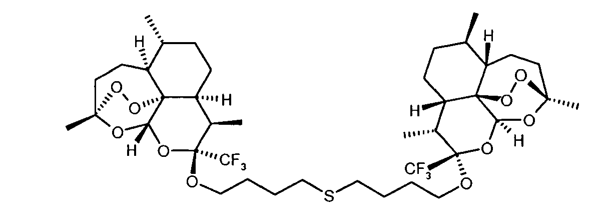

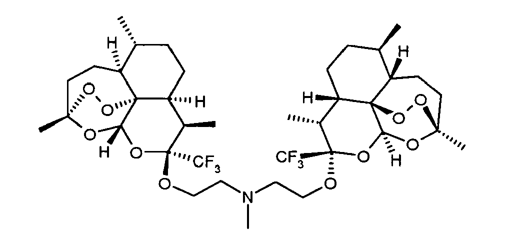

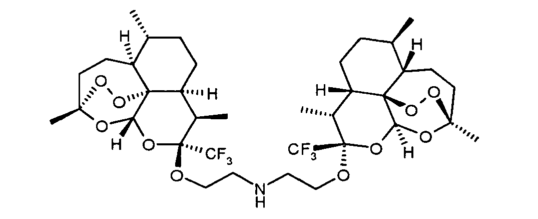

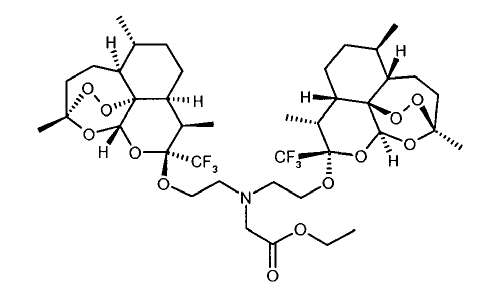

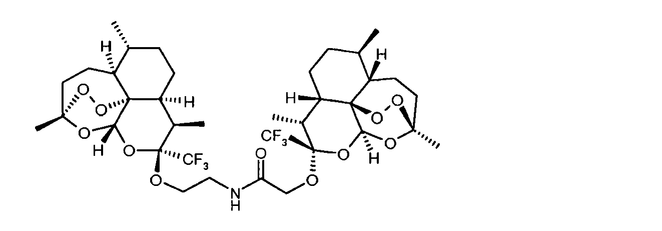

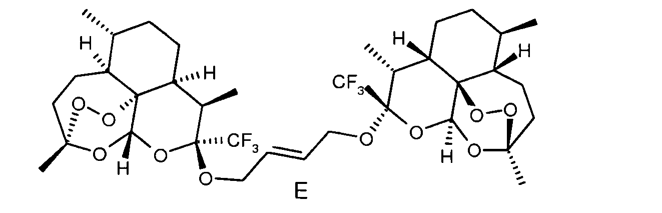

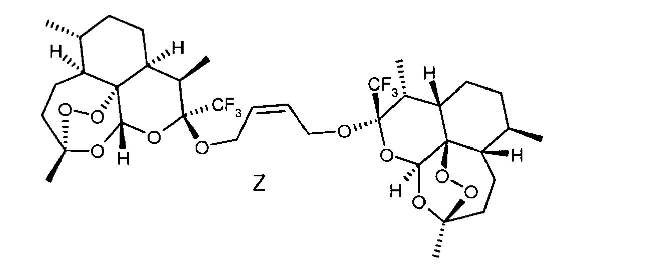

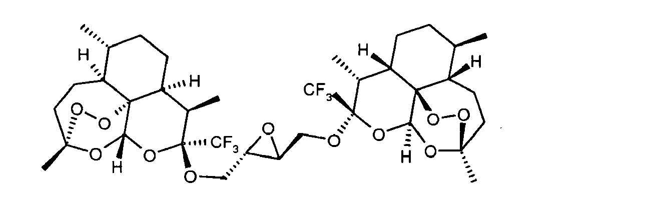

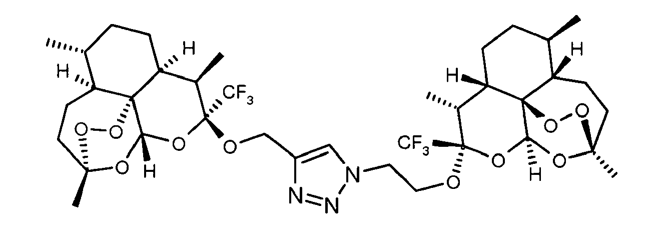

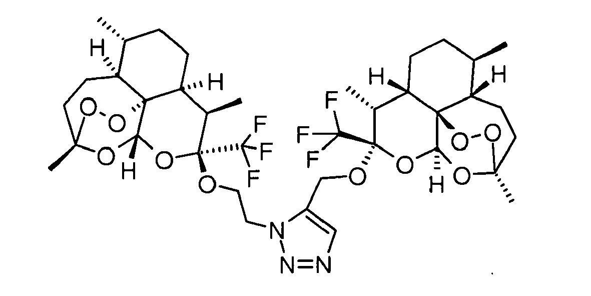

9. Produit selon la revendication 8 **caractérisé en ce que** le produit est sous forme
• non chirale, ou
• racémique, ou
• enrichie en un stéréo-isomère, ou
• enrichie en un énantiomère ;
et **en ce qu'**il est éventuellement salifié.

10. Composition pharmaceutique comprenant un produit selon l'une quelconque des revendications 1 à 9, ou un sel d'addition de ce produit à un acide pharmaceutiquement acceptable, ou encore un hydrate ou un solvat de ce produit.

11. Médicament comprenant un produit selon l'une quelconque des revendications 1 à 9, ou un sel d'addition de ce produit à un acide pharmaceutiquement acceptable, ou encore un hydrate ou un solvat de ce produit.

12. Produit intermédiaire de formule (II) : 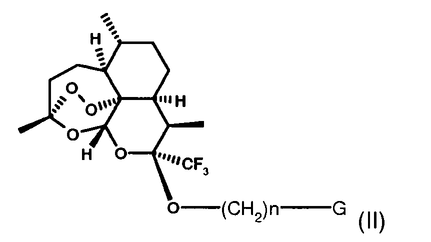 dans laquelle n a pour valeur 1, 2, 3 ou 4 et G représente un atome de brome ou un groupe -N₃.

## Claims

1. Anticancer product of formula (I): 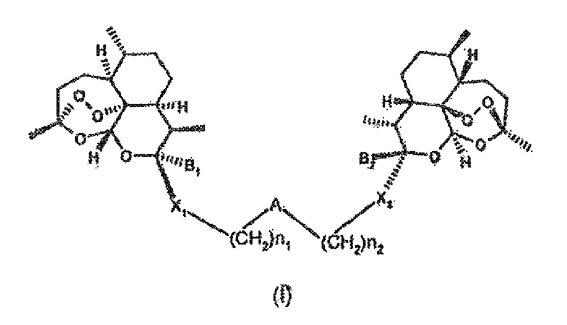 in which:
a) A is a divalent group chosen from -S-, -SO-, -SO₂-, -NH-, -N (CH₂-C(O) O-CH₂-CH₃) - or -N (CH₂-COOH)-, epoxide, (C₁-C₆)alkylene, -NHCO- or 1,2,3-triazole;
b) X₁ and X₂ are identical and are O;
c) n₁ and n₂ are identical or different and have the value 1, 2, 3 or 4;
in the form of a base or of an addition salt with an acid, and in the form of a hydrate or a solvate.

2. Product according to Claim 1, **characterized in that** n₁ and n₂ are identical and have the value 2, 3 or 4.

3. Product according to Claim 1, **characterized in that** A is chosen from -NH-, -N(CH₂-C(O)O-CH₂-CH₃)- or -N(CH₂-COOH)- and that n₁ and n₂ are identical and have the value 2.

4. Product according to Claim 1, **characterized in that** A is chosen from (C₁-C₆) alkenylene or epoxide and that n₁ and n₂ are identical and have the value 1.

5. Product according to Claim 1, **characterized in that** A is chosen from -NHCO- or -1,2,3-triazole and that n₁ and n₂ are different and independently have the value 1 or 2.

6. Product according to any one of Claims 1 to 5, **characterized in that** the product is
• in nonchiral form, or
• in racemic form, or
• in a form enriched in one stereoisomer, or
• in a form enriched in one enantiomer;
and **in that** it is optionally salified.

7. Product according to Claim 1, chosen from the following list:
- (3S,5aS,6R,8aS,9R,10R,12R,12aR,3'S,5'aS,6'R,8'aS,-9'R,10'R,12'R,12'aR)-10,10'-[thiobis(2,1-ethanediyloxy)]bis[decahydro-3,6,9-trimethyl-10-(trifluoromethyl)-3,12-epoxy-12H-pyrano[4,3-j]-1,2-benzodioxepine;
- (3S,5aS,6R,8aS,9R,10R,12R,12aR,3'S,5'aS,6'R,-8'aS,9'R,10'R,12'R,12'aR)-10,10'-[sulfinylbis(2,1-ethanediyloxy)]bis[decahydro-3,6,9-trimethyl-10-(trifluoromethyl)-3,12-epoxy-12H-pyrano[4,3-j]-1,2-benzodioxepine;
- (3S,5aS,6R,8aS,9R,10R,12R,12aR,3'S,5'aS,6'R,-8'aS,9'R,10'R,12'R,12'aR)-10,10'-[sulfonylbis(2,1-ethanediyloxy)]bis[decahydro-3,6,9-trimethyl-10-trifluoromethyl)-3,12-epoxy-12H-pyrano[4,3-j]-1,2-benzodioxepine;
- (3S,5aS,6R,8aS,9R,10R,12R,12aR,3'S,5'aS,6'R,-8'aS,9'R,10'R,12'R,12'aR)-10,10'-[thiobis(3,1-propanediyloxy)]bis[decahydro-3,6,9-trimethyl-10-(trifluoromethyl)-3,12-epoxy-12H-pyrano[4,3-j]-1,2-benzodioxepine;
- (3S,5aS,6R,8aS,9R,10R,12R,12aR,3'S,5'aS,6'R,-8'aS,9'R,10'R,12'R,12'aR)-10,10'-[sulfinylbis(3,1-propanediyloxy)]bis[decahydro-3,6,9-trimethyl-10-(trifluoromethyl)-3,12-epoxy-12H-pyrano[4,3-j]-1,2-benzodioxepine;
- (3S,5aS,6R,8aS,9R,10R,12R,12aR,3'S,5'aS,6'R,-8'aS,9'R,10'R,12'R,12'aR)-10,10'-[sulfonylbis(3,1-propanediyloxy)]bis[decahydro-3,6,9-trimethyl-10-(trifluoromethyl)-3,12-epoxy-12H-pyrano[4,3-j]-1,2-benzodioxepine;
- (3S,5aS,6R,8aS,9R,10R,12R,12aR,3'S,5'aS,6'R,-8'aS,9'R,10'R,12'R,12'aR)-10,10'-[thiobis(4,1-butanediyloxy)]bis[decahydro-3,6,9-trimethyl-10-(trifluoromethyl)-3,12-epoxy-12H-pyrano[4,3-j]-1,2-benzodioxepine;
- 2-[[(3R,5aS,6R,8aS,9R,10R,12R,12aR)-decahydro-3,6,9-trimethyl-10-(trifluoromethyl)-3,12-epoxy-12H-pyrano[4,3-j]-1,2-benzodioxepin-10-yl]oxy]-N-[2-[[(3R,5aS,6R,8aS,9R,10R,12R,12aR)-decahydro-3,6,9-trimethyl-10-(trifluoromethyl)-3,12-epoxy-12H-pyrano[4,3-j]-1,2-benzodioxepin-10-yl]oxy]ethyl]-N-methylethanamine;
- 2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}-N-(2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}ethyl)ethanamine;
- ethyl N,N-bis(2-{[(3S,5aS,6R,8aS,9R,10R,-12R,12aR)-3,6,9-trimethyl-10-(trifluoromethyl)decahydro-3,12-epoxy[1,2]dioxepino[4,3-i]isochromen-10-yl]oxy}ethyl)glycinate;
- N,N-bis(2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}ethyl)glycine;
- 2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}-N-(2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}ethyl)acetamide;
- (3S,5aS,6R,8aS,9R,10R,12R,12aR,3'S,5'aS,6'R,-8'aS,9'R,10'R,12'R,12'aR)-10,10'-[(2E)-but-2-ene-1,4-diylbis(oxy)]bis[3,6,9-trimethyl-10-(trifluoromethyl)decahydro-3,12-epoxy[1,2]dioxepino[4,3-i]isochromene];
- (3S,5aS,6R,8aS,9R,10R,12R,12aR,3'S,5'aS,6'R,-8'aS,9'R,10'R,12'R,12'aR)-10,10'-[(2Z)-but-2-ene-1,4-diylbis(oxy)]bis[3,6,9-trimethyl-10-(trifluoromethyl)decahydro-3,12-epoxy[1,2]dioxepino[4,3-i]isochromene];
- (3S,5aS,6R,8aS,9R,10R,12R,12aR,3'S,5'aS,6'R,-8'aS,9'R,10'R,12'R,12'aR)-10,10'-[(2R,3R)-oxirane-2,3-diylbis(methyleneoxy)]bis[3,6,9-trimethyl-10-(trifluoromethyl)decahydro-3,12-epoxy[1,2]dioxepino[4,3-i]isochromene];
- (1-(2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}ethyl)-4-({[(3S,5aS,6R,8aS,9R,10R,-12R,12aR)-3,6,9-trimethyl-10-(trifluoromethyl)decahydro-3,12-epoxy[1,2]dioxepino[4,3-i]isochromen-10-yl]oxy}methyl)-1H-1,2,3-triazole;
- 1-(2-{[(3S,5aS,6R,8aS,9R,10R,12R,12aR)-3,6,9-trimethyl-10-(trifluoromethyl)decahydro-3,12-epoxy[1,2]dioxepino[4,3-i]isochromen-10-yl]oxy}ethyl)-5-({[(3S,5aS,6R,8aS,9R,10R,-12R,12aR)-3,6,9-trimethyl-10-(trifluoromethyl)decahydro-3,12-epoxy[1,2]dioxepino[4,3-i]isochromen-10-yl]oxy}ethyl)-1H-1,2,3-triazole.

8. Product according to Claim 1, chosen from the following list:

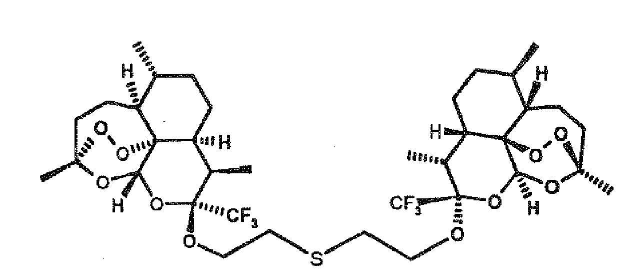

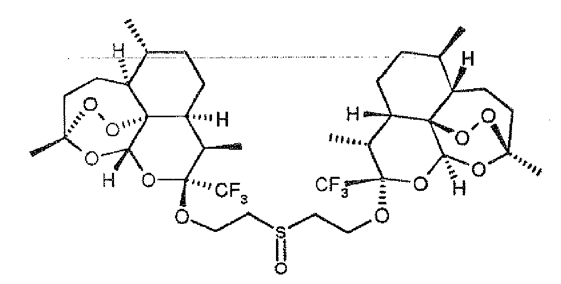

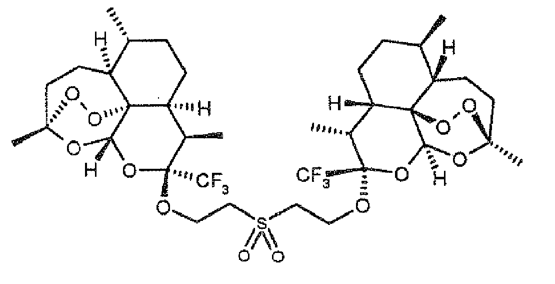

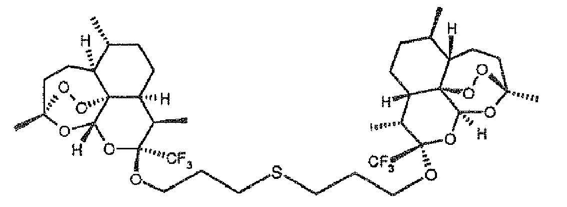

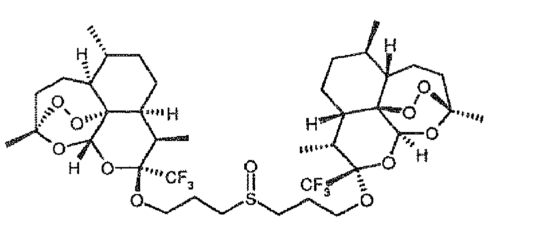

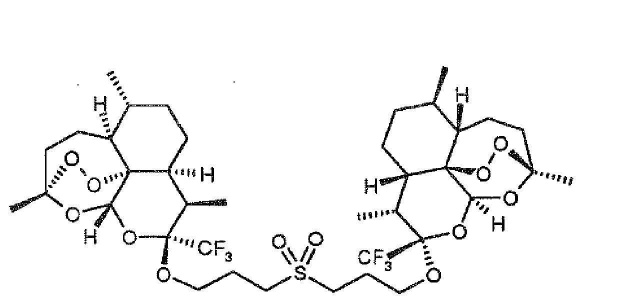

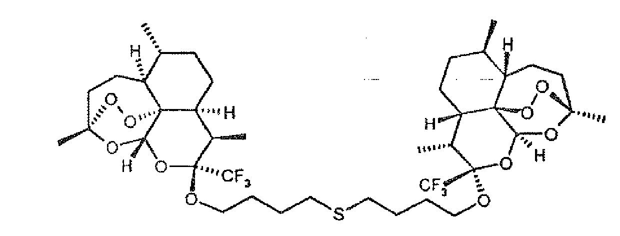

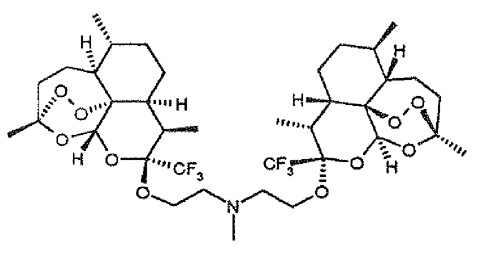

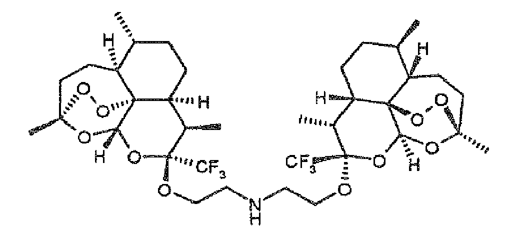

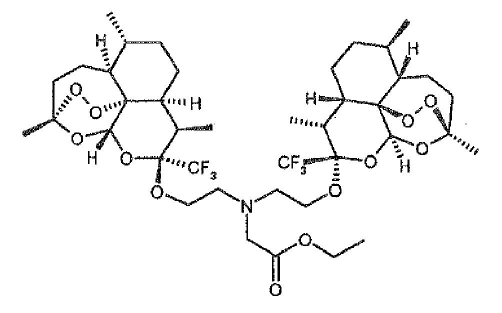

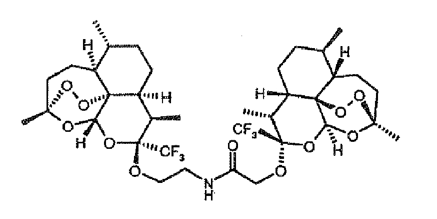

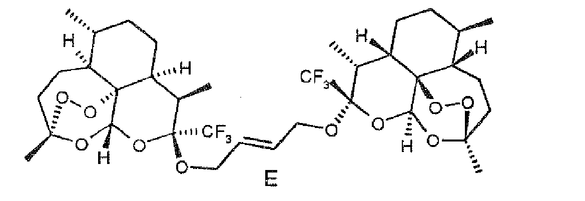

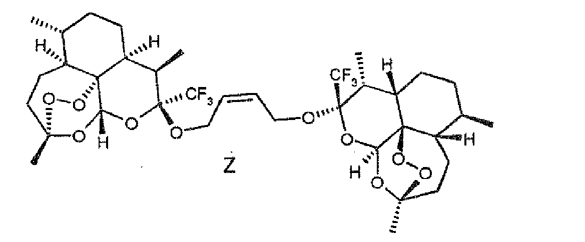

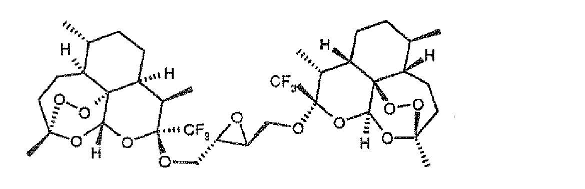

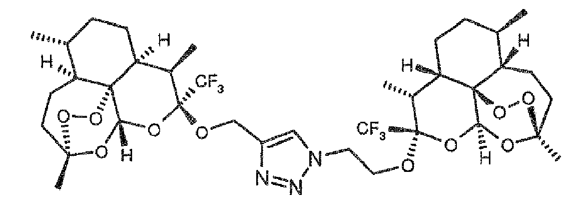

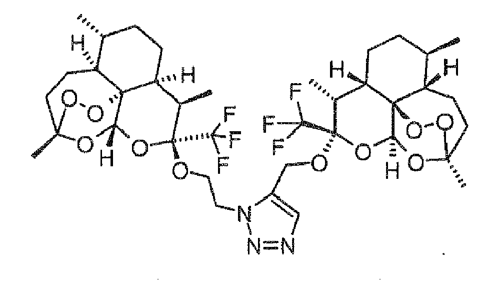

9. Product according to Claim 8, **characterized in that** the product is
• in nonchiral form, or
• in racemic form, or
• in a form enriched in one stereoisomer, or
• in a form enriched in one enantiomer;
and **in that** it is optionally salified.

10. Pharmaceutical composition comprising a product according to any one of Claims 1 to 9, or an addition salt of this product with a pharmaceutically acceptable acid, or a hydrate or a solvate of this product.

11. Medicament comprising a product according to any one of Claims 1 to 9, or an addition salt of this product with a pharmaceutically acceptable acid, or a hydrtae or a solvate of this product.

12. Intermediate product of formula (II): 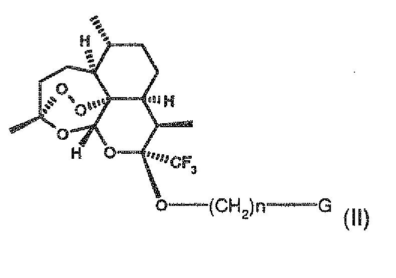 in which n has the value 1, 2, 3 or 4 and G represents a bromine atom or an -N3 group.

## Patentansprüche

1. Antikrebsprodukt der Formel (I): 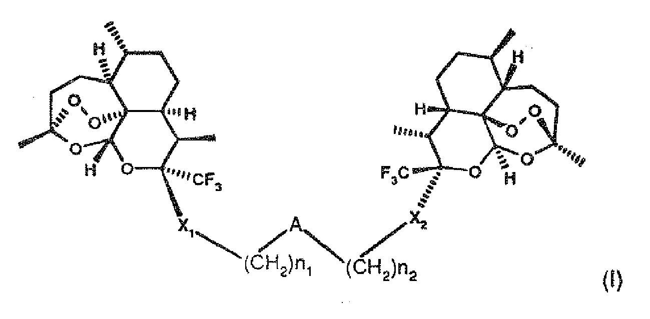 worin:
a). A für eine zweiwertige Gruppe steht, die unter -S-, -SO-, -SO₂-, -NH-, -N(CH₂-C(O)O-CH₂-CH₃)- oder -N (CH₂-COOH) -, Epoxid, (C₁-C₆) -Alkenylen, -NHCO- oder 1,2,3-Triazol ausgewählt ist;
b). X₁ und X₂ gleich sind und für O stehen;
c). n₁ und n₂ gleich oder verschieden sind und einen Wert von 1, 2, 3 oder 4 aufweisen;
in Basen- oder Säureadditionssalzform sowie in Hydrat- oder Solvatform.

2. Produkt nach Anspruch 1, **dadurch gekennzeichnet, daß** n₁ und n₂ gleich sind und einen Wert von 2, 3 oder 4 aufweisen.

3. Produkt nach Anspruch 1, **dadurch gekennzeichnet, daß** A unter -NH-, -N(CH₂-C(O)O-CH₂-CH₃) - oder -N(CH₂-COOH)- ausgewählt ist und n₁ und n₂ gleich sind und einen Wert von 2 aufweisen.

4. Produkt nach Anspruch 1, **dadurch gekennzeichnet, daß** A unter (C₁-C₆)-Alkenylen oder Epoxid ausgewählt ist und n₁ und n₂ gleich sind und einen Wert von 1 aufweisen.

5. Produkt nach Anspruch 1, **dadurch gekennzeichnet, daß** A unter -NHCO- oder 1,2,3-Triazol ausgewählt ist und n₁ und n₂ verschieden sind und unabhängig voneinander einen Wert von 1 oder 2 aufweisen.

6. Produkt nach einem der Ansprüche 1 bis 5, **dadurch gekennzeichnet, daß** das Produkt
• in achiraler Form oder
• in racemischer Form oder
• in mit einem Stereoisomer angereicherter Form oder
• in mit einem Enantiomer angereicherter Form
vorliegt und gegebenenfalls versalzt ist.

7. Produkt nach Anspruch 1, ausgewählt aus der folgenden Liste:
• (3S,5aS,6R,8aS,9R,10R,12R,12aR,3'S,5'aS,6'R,-8'aS,9'R,10'R,12'R,12'aR)-10,10'-[Thiobis(2,1-ethandiyloxy)]bis[decahydro-3,6,9-trimethyl-10-(trifluormethyl)-3,12-epoxy-12H-pyrano[4,3-j]-1,2-benzodioxepin;
• (3S,5aS,6R,8aS,9R,10R,12R,12aR,3'S,5'aS,6'R,-8'aS,9'R,10'R,12'R,12'aR)-10,10'-[Sulfinylbis-(2,1-ethandiyloxy)]bis[decahydro-3,6,9-trimethyl-10-(trifluormethyl)-3,12-epoxy-12H-pyrano[4,3-j]-1,2-benzodioxepin;
• (3S,5aS,6R,8aS,9R,10R,12R,12aR,3'S,5'aS,6'R,-8'aS,9'R,10'R,12'R,12'aR)-10,10'-[Sulfonylbis-(2,1-ethandiyloxy)]bis[decahydro-3,6,9-trimethyl-10-(trifluormethyl)-3,12-epoxy-12H-pyrano[4,3-j]-1,2-benzodioxepin;
• (3S,5aS,6R,8aS,9R,10R,12R,12aR,3'S,5'aS,6'R,-8'aS,9'R,10'R,12'R,12'aR)-10,10'-[Thiobis(3,1-propandiyloxy)]bis[decahydro-3,6,9-trimethyl-10-(trifluormethyl)-3,12-epoxy-12H-pyrano[4,3-j]-1,2-benzodioxepin;
• (3S,5aS,6R,8aS,9R,10R,12R,12aR,3'S,5'aS,6'R,-8'aS,9'R,10'R,12'R,12'aR)-10,10'-[Sulfinylbis-(3,1-propandiyloxy)]bis[decahydro-3,6,9-trimethyl-10-(trifluormethyl)-3,12-epoxy-12H-pyrano[4,3-j]-1,2-benzodioxepin;
• (3S,5aS,6R,8aS,9R,10R,12R,12aR,3'S,5'aS,6'R,-8'aS,9'R,10'R,12'R,12'aR)-10,10'-[Sulfonylbis-(3,1-propandiyloxy)]bis[decahydro-3,6,9-trimethyl-10-(trifluormethyl)-3,12-epoxy-12H-pyrano[4,3-j]-1,2-benzodioxepin;
• (3S,5aS,6R,8aS,9R,10R,12R,12aR,3'S,5'aS,6'R,-8'aS,9'R,10'R,12'R,12'aR)-10,10'-[Thiobis(4,1-butandiyloxy)]bis[decahydro-3,6,9-trimethyl-10-(trifluormethyl)-3,12-epoxy-12H-pyrano[4,3-j]-1,2-benzodioxepin;
• 2-[[(3R,5aS,6R,8aS,9R,10R,12R,12aR)-Decahydro-3,6,9-trimethyl-10-(trifluormethyl)-3,12-epoxy-12H-pyrano[4,3-j]-1,2-benzodioxepin-10-yl]oxy]-N-[2-[[(3R,5aS,6R,8aS,9R,10R,12R,12aR)-decahydro-3,6,9-trimethyl-10-(trifluormethyl)-3,12-epoxy-12H-pyrano[4,3-j]-1,2-benzodioxepin-10-yl]oxy]ethyl]-N-methylethanamin;
• 2-{[(3S,5aS,6R,8aS,9R,10R,12R,12aR)-3,6,9-Trimethyl-10-(trifluormethyl)decahydro-3,12-epoxy[1,2]dioxepino[4,3-i]isochromen-10-yl]oxy}-N-(2-{[(3S,5aS,6R,8aS,9R,10R,12R,12aR)-3,6,9-trimethyl-10-(trifluormethyl)decahydro-3,12-epoxy[1,2]dioxepino[4,3-i]isochromen-10-yl]oxy}ethyl)ethanamin;
• N,N-Bis(2-{[(3S,5aS,6R,8aS,9R,10R,12R,12aR)-3,6,9-trimethyl-10-(trifluormethyl)decahydro-3,12-epoxy[1,2]dioxepino[4,3-i]isochromen-10-yl]oxy}ethyl)glycinethylester;
• N,N-Bis(2-{[(3S,5aS,6R,8aS,9R,10R,12R,12aR)-3,6,9-trimethyl-10-(trifluormethyl)decahydro-3,12-epoxy[1,2]dioxepino[4,3-i]isochromen-10-yl]oxy}ethyl)glycin;
• 2-{[(3S,5aS,6R,8aS,9R,10R,12R,12aR)-3,6,9-Trimethyl-10-(trifluormethyl)decahydro-3,12-epoxy[1,2]dioxepino[4,3-i]isochromen-10-yl]oxy}-N-(2-{[(3S,5aS,6R,8aS,9R,10R,12R,12aR)-3,6,9-trimethyl-10-(trifluormethyl)decahydro-3,12-epoxy[1,2]dioxepino[4,3-i]isochromen-10-yl]oxy}ethyl)acetamid;
• (3S,5aS,6R,8aS,9R,10R,12R,12aR,3'S,5'aS,6'R,-8'aS,9'R,10'R,12'R,12'aR)-10,10'-[(2E)-But-2-en-1,4-diylbis(oxy)]bis[3,6,9-trimethyl-10-(trifluormethyl)decahydro-3,12-epoxy[1,2]dioxepino-[4,3-i]isochromen];
• (3S,5aS,6R,8aS,9R,10R,12R,12aR,3'S,5'aS,6'R,-8'aS,9'R,10'R,12'R,12'aR)-10,10'-[(2Z)-But-2-en-1,4-diylbis(oxy)]bis[3,6,9-trimethyl-10-(trifluormethyl)decahydro-3,12-epoxy[1,2]dioxepino-[4,3-i]isochromen];
• (3S,5aS,6R,8aS,9R,10R,12R,12aR,3'S,5'aS,6'R,-8'aS,9'R,10'R,12'R,12'aR)-10,10'-[(2R,3R)-Oxiran-2,3-diylbis(methylenoxy)]bis[3,6,9-trimethyl-10-(trifluormethyl)decahydro-3,12-epoxy-[1,2]dioxepino[4,3-i]isochromen];
• (1-(2-{[(3S,5aS,6R,8aS,9R,10R,12R,12aR)-3,6,9-Trimethyl-10-(trifluormethyl)decahydro-3,12-epoxy[1,2]dioxepino[4,3-i]isochromen-10-yl]oxy}ethyl)-4-({[(3S,5aS,6R,8aS,9R,10R,12R,12aR)-3,6,9-trimethyl-10-(trifluormethyl)decahydro-3,12-epoxy[1,2]dioxepino[4,3-i]isochromen-10-yl]oxy}methyl)-1H-1,2,3-triazol;
• 1-(2-{[(3S,5aS,6R,8aS,9R,10R,12R,12aR)-3,6,9-Trimethyl-10-(trifluormethyl)decahydro-3,12-epoxy[1,2]dioxepino[4,3-i]isochromen-10-yl]oxy}ethyl)-5-({[(3S,5aS,6R,8aS,9R,10R,12R,12aR)-3,6,9-trimethyl-10-(trifluormethyl)decahydro-3,12-epoxy[1,2]dioxepino[4,3-i]isochromen-10-yl]oxy}ethyl)-1H-1,2,3-triazol.

8. Produkt nach Anspruch 1, ausgewählt aus der folgenden Liste:

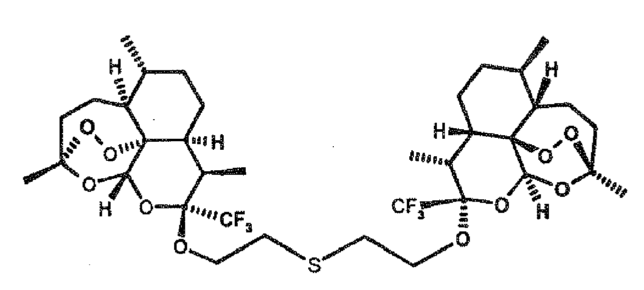

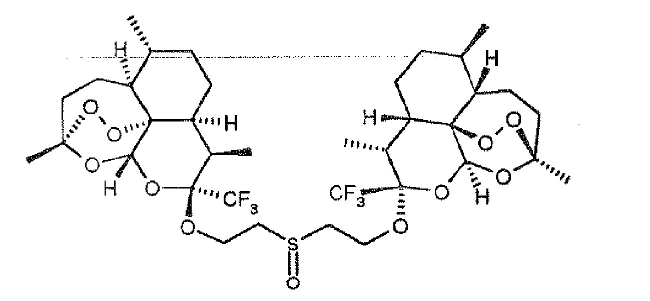

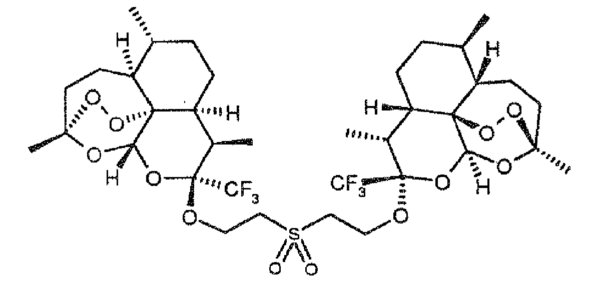

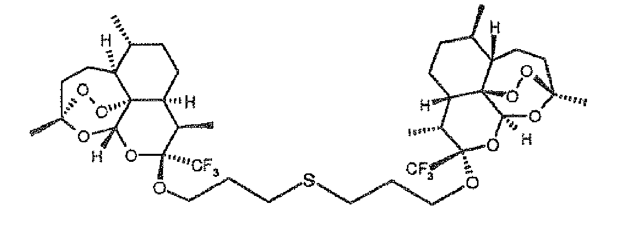

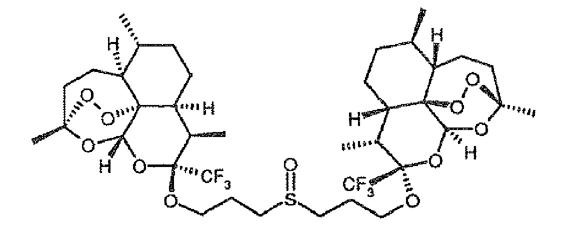

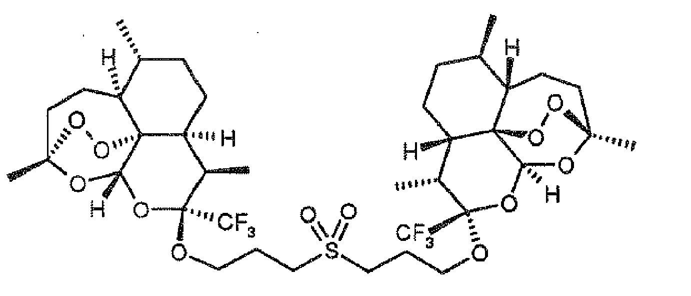

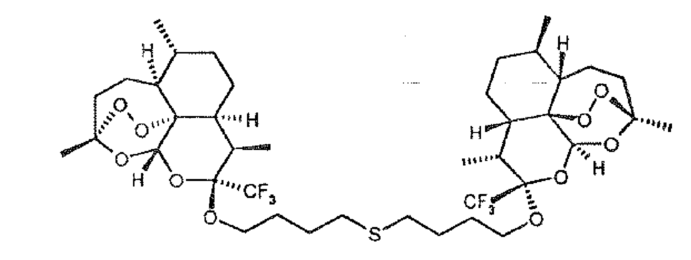

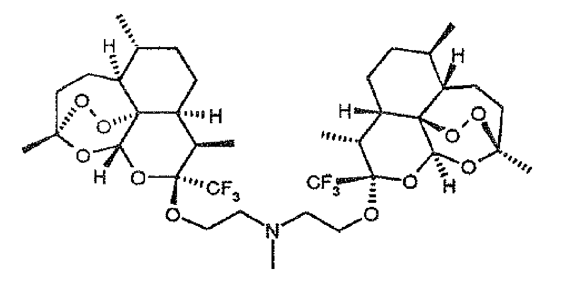

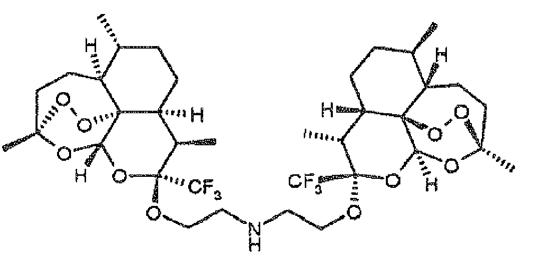

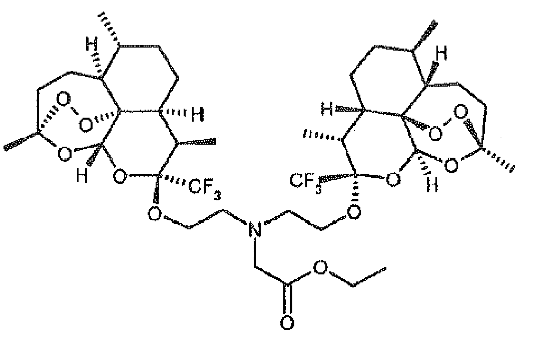

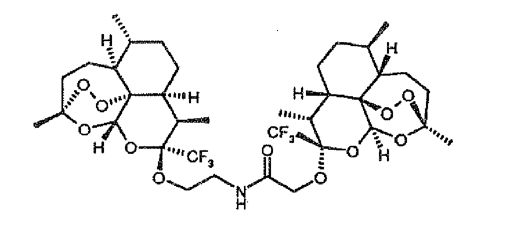

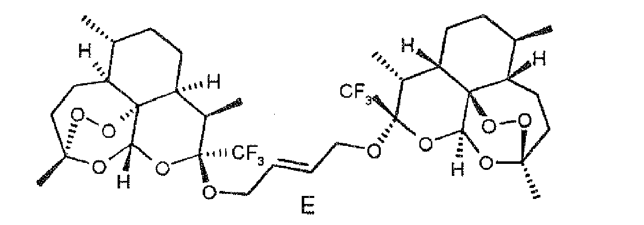

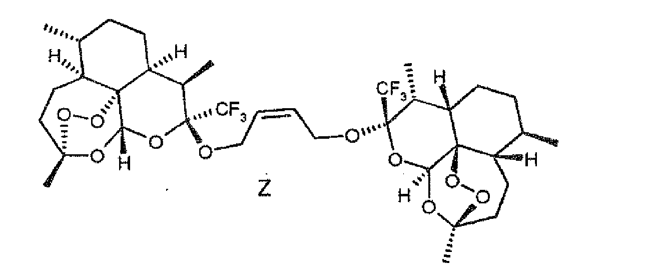

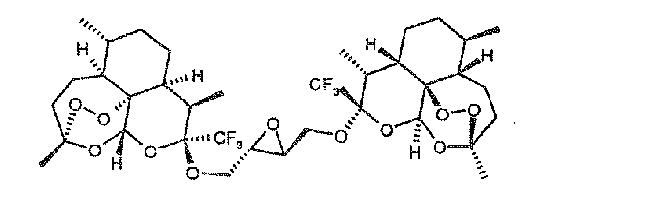

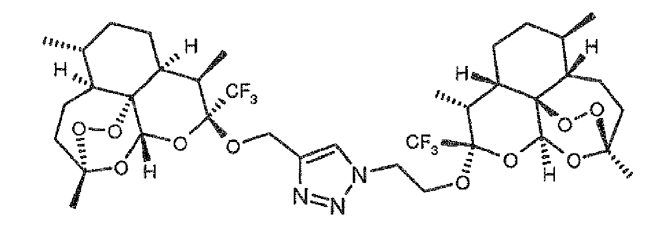

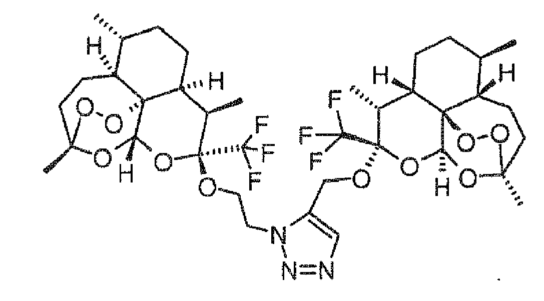

9. Produkt nach Anspruch 8, **dadurch gekennzeichnet, daß** das Produkt
• in achiraler Form oder
• in racemischer Form oder
• in mit einem Stereoisomer angereicherter Form oder
• in mit einem Enantiomer angereicherter Form
vorliegt und gegebenenfalls versalzt ist.

10. Pharmazeutische Zusammensetzung, umfassend ein Produkt nach einem der Ansprüche 1 bis 9 oder ein Additionssalz dieses Produkts mit einer pharmazeutisch unbedenklichen Säure oder auch ein Hydrat oder ein Solvat dieses Produkts.

11. Medikament, umfassend ein Produkt nach einem der Ansprüche 1 bis 9 oder ein Additionssalz dieses Produkts mit einer pharmazeutisch unbedenklichen Säure oder auch ein Hydrat oder ein Solvat dieses Produkts.

12. Zwischenprodukt der Formel (II): 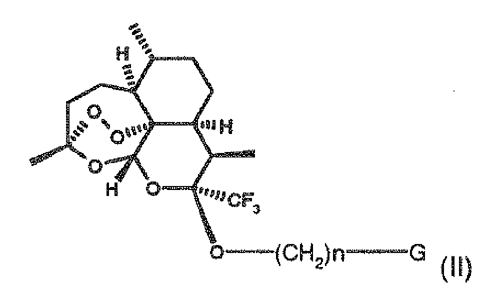 worin n einen Wert von 1, 2, 3 oder 4 aufweist und G für ein Bromatom oder eine -N₃-Gruppe steht.
